(19) Europäisches Patentamt
European Patent Office
Office européen des brevets

(11) Veröffentlichungsnummer: **0 307 872 A2**

(12) **EUROPÄISCHE PATENTANMELDUNG**

(21) Anmeldenummer: **88114962.9**

(22) Anmeldetag: **14.09.88**

(51) Int. Cl.4: **C07K 5/06 , A61K 37/02 , C07D 317/40**

Patentansprüche für folgende Vertragsstaaten: ES + GR.

(30) Priorität: **16.09.87 DE 3731085**

(43) Veröffentlichungstag der Anmeldung: **22.03.89 Patentblatt 89/12**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim (Taunus)(DE)**
Erfinder: **Urbach, Hansjörg, Dr.**
**Le Lavandoustrasse 41**
**D-6242 Kronberg/Taunus(DE)**
Erfinder: **Becker, Reinhold, Dr.**
**Humboldtstrasse 17**
**D-6200 Wiesbaden(DE)**
Erfinder: **Hock, Franz, Dr.**
**Altstadt 19**
**D-6110 Dieburg(DE)**

(54) **Neue Aminosäureester, Verfahren zu ihrer Herstellung, sie enthaltende Arzneimittel und deren Verwendung.**

(57) Die Erfindung betrifft Aminosäureester der Formel I

$$R^3OOC - \underset{R^4}{\overset{*}{CH}} - \underset{R^5}{N} - \underset{O}{\overset{}{\underset{\|}{C}}} - \underset{R^1}{\overset{*}{CH}} - NH - \underset{COOR_2}{\overset{*}{CH}} - (CH_2)_n\text{-}R \qquad (I)$$

worin n = 1 oder 2 ist, R, $R^1$, $R^2$ und $R^3$ Wasserstoff oder einen definierten Rest bedeuten, $R^4$ zusammen mit $R^5$ und den sie tragenden Atomen ein heterocyclisches Ringsystem bilden, Verfahren zu ihrer Herstellung, sie enthaltende Mittel und deren Verwendung.

EP 0 307 872 A2

**Neue Aminosäureester, Verfahren zu ihrer Herstellung, sie enthaltende Arzneimittel und deren Verwendung**

Die Erfindung betrifft Aminosäureester der Formel I

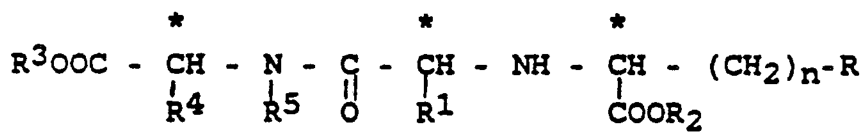
(I)

in welcher
n = 1 oder 2 ist,
R = Wasserstoff,
einen gegebenenfalls substituierten aliphatischen Rest mit 1 - 21 C-Atomen,
einen gegebenenfalls substituierten alicyclishen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclischaliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
R' Wasserstoff,
einen gegebenenfalls substituierten aliphatischen Rest mit 1 - 21 C-Atomen,
einen gegebenenfalls substituierten alicylishcen Rest mit 3 - 20 C-Atomen,
einen gegebenenfalls substituierten alicyclishaliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden $\alpha$-Aminosäure bedeutet,

$R^2$ und $R^3$ gleich oder verschieden sind und Wasserstoff,
einen gegebenenfalls substituierten aliphatischen Rest mit 1 - 21 C-Atomen, einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

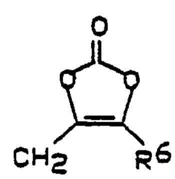

worin $R^5$ Wasserstoff, ein aliphatischer Rest mit 1 - 6 C-Atomen oder ein gegebenenfalls substituierter aromatischer Rest mit 6 - 12 C-Atomen ist,
bedeuten,
wobei mindestens einer der Reste $R^2$ oder $R^3$ einen Rest der Formel

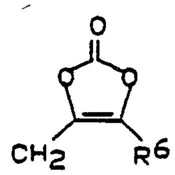

bedeutet
und
$R^4$ und $R^5$ zusammen mit den sie tragenden Atomen ein bi-, oder tricyclisches heterocyclisches Ringsystem mit 5 bis 15 Ring-C-Atomen bilden,
sowie deren physiologisch verträgliche Salze mit Säuren und Basen.

Unter einem gegebenenfalls substituierten aliphatischen Rest versteht man einen aliphatischen acyclischen Rest, d.h. einen Rest mit einer offenen, geraden oder verzweigten Kohlenstoffkette, wie beispielsweise Alkyl, Alkenyl, Alkinyl und entsprechende mehrfach ungesättigte Reste. Er ist unsubstituiert oder, wie unten beispielsweise bei Carboxy, Carbamoyl, Aminoalkyl, Alkanoylaminoalkyl, Alkoxycarbonylaminoalkyl, Arylalkoxycarbonylaminoalkyl, Arylalkylaminoalkyl, Alkylaminoalkyl, Dialkylaminoalkyl, Alkylthioalkyl, Arylthioalkyl, Carboxyalkyl, Carbamoylalkyl, Alkoxycarbonylalkyl, Alkanoyloxyalkyl, Alkoxycarbonyloxyalkyl, Aroyloxyalkyl oder Aryloxycarbonyloxyalkyl beschrieben, monosubstituiert, vorzugsweise ($C_1$-$C_5$)-Alkanoyloxy-($C_1$-$C_8$)alkyl, insbesondere Acetoxymethyl, Propionyloxymethyl, Isopropionyloxymethyl, n-Butyryloxymethyl, Pivaloyloxymethyl, Isovaleroyloxymethyl, 1-Acetoxyethyl, 1-n-Propionyloxyethyl, 1-Acetoxypropyl, oder ($C_1$-$C_6$)-Alkoxycarbonyloxy-($C_1$-$C_8$)-alkyl, insbesondere 1-Methoxycarbonyloxyethyl, 1-Ethoxycarbonyloxyethyl, 1-Isopropoxycarbonyloxyethyl, 1-(Cyclohexyloxycarbonyloxy)-ethyl, Methoxycarbonyloxymethyl.

Ein gegebenenfalls substituierter alicyclischer Rest und der über eine offene Kohlenstoffkette gebundene entsprechende gegebenenfalls substituierte alicyclisch-aliphatische Rest ist ein vorzugsweise mono- bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Er ist vorzugsweise unsubstituiert. Mehrere Ringe als Komponenten eines solchen Restes sind kondensiert, spiroverknüpft oder isoliert. Beispiele für solche Reste sind Cycloalkyl, Cycloalkenyl, Cycloalkylalkyl, Bicycloalkyl, Tricycloalkyl und von mono-, bi- oder oligocyclischen Terpenen abgeleitete Reste, wie Menthyl, Isomenthyl, Bornyl, Caranyl, Epibornyl, Epiisobornyl, Isobornyl, Norbornyl, Neomenthyl, Neoisomenthyl, Pinanyl, Thujanyl; sie sind vorzugsweise unsubstituiert (aliphatische Seitenketten sind nach vorliegender Definition keine Substituenten).

Ein gegebenenfalls substituierter aromatischer Rest ist vorzugsweise Aryl wie Phenyl, Biphenylyl oder Naphthyl, das gegebenenfalls wie unten bei Aryl angegeben mono-, di- oder trisubstituiert ist. Von Aryl abgeleitete Reste, wie Aralkyl, Aryloxy, Arylthio oder Aroyl, vorzugsweise Benzoyl, können wie Aryl substituiert sein.

Ein gegebenenfalls substituierter heteroaromatischer Rest ist vorzugsweise ein aromatischer mono- bzw. bicyclischer Heterocyclen-Rest mit 5 bis 7 bzw. 8 bis 12, bevorzugt bis 10 Ringatomen, wovon 1 bis 2 Ringatome Schwefel- oder Sauerstoffatome und/oder wovon 1 bis 4 Ringatome Stickstoffatome darstellen, wie beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl. Diese Reste können auch teilweise oder vollständig hydriert sein. Ein heteroaromatischer Rest und der entsprechende heteroaromatisch-aliphatische Rest kann wie unten definiert, substituiert sein.

Unter einem gegebenenfalls substituierten araliphatischen Rest werden insbesondere Phthalidyl und Arylalkyl, Diarylalkyl, Indanyl oder Fluorenyl verstanden, worin Aryl wie oben definiert ist und in der dort angegebenen Weise substituiert sein kann.

$R^4$ und $R^5$ können mit den diese tragenden Atomen ein mono-, bi- oder tricyclisches heterocyclisches Ringsystem mit 3 bis 15 Ring C-Atomen bilden, das im Ring vorzugsweise bis zu 2 S-Atome und bis zu 2 N-Atome, insbesondere bis zu 1 S-Atom aufweist.

Als solche Ringsysteme kommen insbesondere jene aus der folgenden Gruppe in Betracht:

Tetrahydroisochinolin (A); Decahydroisochinolin (B); Octahydroindol (C); Octahydrocyclopenta-[b]pyrrol (D); 2-Azaspiro[4.5]decan (E); 2-Azaspiro[4.4]-nonan (F); Spiro[(bicyclo[2.2.1]heptan)-2,3′-pyrrolidin] (G); Spiro[(bicyclo[2.2.2]octan)-2,3′-pyrrolidin] (H); 2-Azatricyclo[4.3.0.1$^{6,9}$]decan (I); Decahydrocyclohepta-[b]-pyrrol (J); Hexahydrocyclopropa[b]pyrrol (K); Octahydroisoindol (L); Octahydrocyclopenta [c]pyrrol (M); 2,3,3a,4,5,7a-Hexahydroindol (N); 1,2,3,3a,4,6a-Hexahydrocyclopenta[b]pyrrol (O); die alle gegebenenfalls substituiert sein können.

Tetrahydroisochinolin (A) kann z.B. im Arylteil bis zu 2 ($C_1$-$C_6$)-Alkoxyreste, vorzugsweise Methoxyreste tragen. Entsprechendes gilt für die anderen Ringsystem. Bevorzugt sind jedoch die unsubstituierten Systeme.

Die in Betracht kommenden cyclischen Aminosäureester weisen die folgenden Strukturformeln auf:

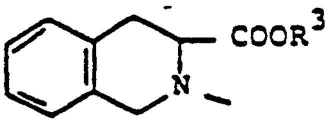
A

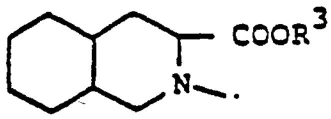
B

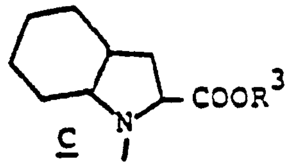
C

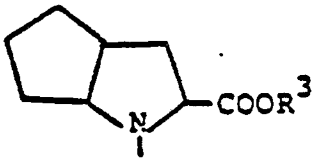
D

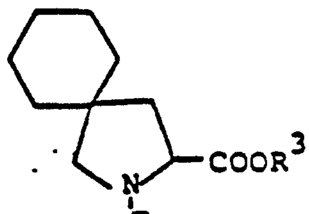
E

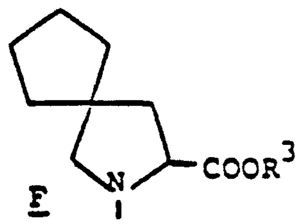
F

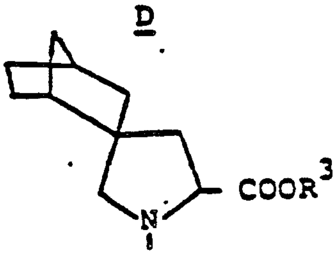
G

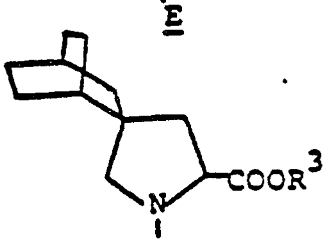
H

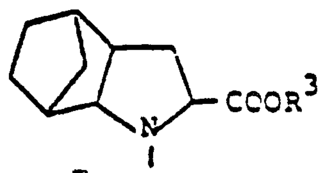
I

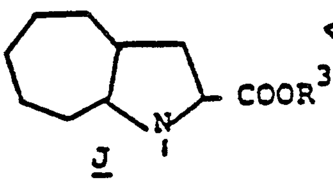
J

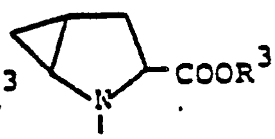
K

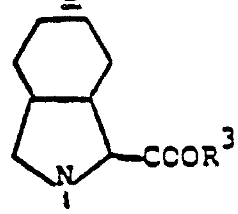
L

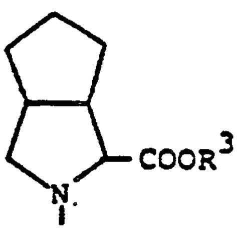
M

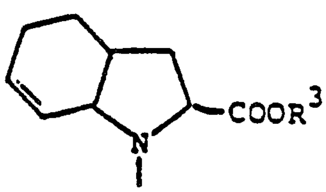
N

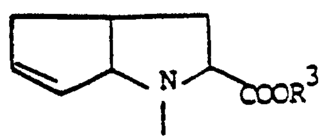
O

Besonders bevorzugt sind die Ringsysteme A, C, D und H in welchen das die $COOR^3$-Gruppe tragende C-Atom vorzugsweise die S-Konfiguration aufweist.

Natürlich vorkommende α-Aminosäuren sind beispielsweise Ala, Ser, Thr, Val, Leu, Ile, Asp, Asn, Glu, Gln, Arg, Lys, Hyl, Orn, Cit, Tyr, Phe, Trp und His.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen bevorzugt ($C_1$-$C_6$)-Alkyl, insbesondere Methyl oder Ethyl in Frage.

Die Verbindungen der Formel I weisen asymmetrische Kohlenstoffatome auf und können daher als Enantiomere und Diastereomere auftreten. Die Erfindung umfaßt sowohl die reinen Enantiomeren als auch die Racemate.

Bei den Verbindungen, die mehrere chirale Atome besitzen, kommen alle möglichen Diastereomere als

Racemate oder Enantiomere, oder Gemische verschiedener Diastereomere in Betracht. Die Racemate können nach gebräuchlichen Methoden, zum Beispiel durch Salzbildung mit optisch aktiven Säuren wie Camphersulfonsäure oder Di-benzoylweinsäure, fraktionierte Kristallisation und anschließende Freisetzung der Basen aus ihren Salzen, oder durch Derivatisierung mit geeigneten optisch aktiven Reagenzien, Trennung der diastereomeren Derivate durch fraktionierte Kristallisation oder Chromatographie an Kieselgel oder Aluminiumoxid und Rückspaltung in die Enantiomeren aufgetrennt werden. Die Diastereomeren können nach gebräuchlichen Methoden, wie fraktionierte Kristallisation oder Chromatographie an Säulen, getrennt werden.

Bevorzugt sind Verbindungen der Formel I, in welchen

a) n = 1 oder 2 ist

b) R

1. Wasserstoff bedeutet,

2. Alkyl mit 1 - 18 C-Atomen bedeutet,

3. einen aliphatischen acyclischen Rest der Formel $C_aH_{2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;

5. Aryl mit 6 - 12 C-Atomen bedeutet,

das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann;

6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter b) 5. beschrieben substituiert sein können; oder

7. Alkoxy mit 1 - 4 C-Atomen;

8. Aryloxy mit 6 - 12 C-Atomen,

das wie unter b) 5. beschrieben substituiert sein kann;

9. mono- bzw. bicyclisches Heteroaryloxy oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b) 5. beschrieben substituiert sein können.

10. Amino-($C_1$-$C_8$)-alkyl;

11. ($C_1$-$C_4$)-Alkanoylamino-($C_1$-$C_8$)-alkyl;

12. ($C_7$-$C_{13}$)-Aroylamino-($C_1$-$C_8$)-alkyl;

13. ($C_1$-$C_4$)-Alkoxy-carbonylamino-($C_1$-$C_8$)-alkyl;

14. ($C_6$-$C_{12}$)-Aryl-($C_1$-$C_4$)-alkoxycarbonylamino-($C_1$-$C_8$)-alkyl;

15. ($C_6$-$C_{12}$)-Aryl-($C_1$-$C_4$)-alkylamino-($C_1$-$C_8$)-alkyl;

16. ($C_1$-$C_4$)-Alkylamino-($C_1$-$C_8$)-alkyl;

17. Di-($C_1$-$C_4$)-alkylamino-($C_1$-$C_8$)-alkyl;

18. Guanidino-($C_1$-$C_8$)-alkyl;

19. Imidazolyl;

20. Indolyl;

21. ($C_1$-$C_4$)-Alkylthio;

22. falls n = 2 ist, ($C_1$-$C_4$)-Alkylthio-($C_1$-$C_8$)-alkyl;

23. ($C_6$-$C_{12}$)-Arylthio-($C_1$-$C_8$)-alkyl;

das im Arylteil wie unter b) 5. beschrieben, substituiert sein kann,

24. ($C_6$-$C_{12}$)-Aryl-($C_1$-$C_8$)-alkylthio,

das im Arylteil wie unter b) 5. beschrieben substituiert sein kann;

25. falls n = 2 ist, Carboxy-($C_1$-$C_8$)-alkyl;

26. Carboxy;

27. Carbamoyl;

28. falls n = 2 ist, Carbamoyl-($C_1$-$C_8$)-alkyl;

29. ($C_1$-$C_4$)-Alkoxy-carbonyl-($C_1$-$C_8$)-alkyl;

30. falls n = 2 ist, ($C_6$-$C_{12}$)-Aryloxy-($C_1$-$C_8$)-alkyl,

das im Arylteil unter b) 5. beschrieben substituiert sein kann; oder

31. $(C_6-C_{12})$-Aryl-$(C_1-C_8)$-alkoxy,
das im Arylteil unter b) 5. beschrieben substituiert sein kann, bedeutet;

c) $R^1$

1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;

5. Aryl mit 6 - 12 C-Atomen,
das wie unter b) 5. beschrieben substituiert sein kann;

6. $(C_6-C_{12})$-Aryl-$(C_1-C_8)$-alkyl oder $(C_7-C_{13})$-Aroyl-$(C_1-C_8)$-alkyl,
die beide wie Aryl unter b) 5. beschrieben substituiert sein können;

7. mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel- oder Sauerstoffatome und/oder 1 bis 4 Ringatome Stickstoffatome darstellen, die im Heteroaryl wie bei Aryl unter b) 5. beschrieben substituiert sein können; oder

8. falls von c) 1. -7. noch nicht umfaßt, die gegebenenfalls geschützte Seitenkette einer natürlich vorkommenden α-Aminosäure der Formel $R^1$-$CH(NH_2)$-$COOH$ bedeuten;

d) $R^2$ und $R^3$ gleich oder verschieden sind und

1. Wasserstoff bedeutet;
2. Alkyl mit 1 - 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Di-$(C_1-C_4)$-alkylamino-$(C_1-C_8)$-alkyl;
6. $(C_1-C_5)$-Alkanoyloxy-$(C_1-C_8)$-alkyl;
7. $(C_1-C_6)$-Alkoxy-carbonyloxy-$(C_1-C_8)$-alkyl;
8. $(C_7-C_{13})$-Aroyloxy-$(C_1-C_8)$-alkyl;
9. $(C_6-C_{12})$-Aryloxycarbonyloxy$(C_1-C_8)$-alkyl;
10. Aryl mit 6 - 12 C-Atomen;
11. $(C_7-C_{20})$-Aralkyl;
12. Phthalidyl; oder
13. einen Rest der Formel

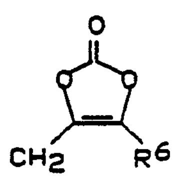

bedeuten, worin $R^6$ Wasserstoff, $(C_1-C_5)$-Alkyl oder Aryl mit 6 - 12 C-Atomen ist,
wobei die unter d) 8., 9., 10., 11. und 12. genannten Reste wie unter b) 5. beschrieben im Arylteil substituiert sein können; und wobei mindestens einer der Reste $R^2$ oder $R^3$ einen Rest der Formel

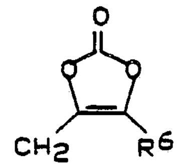

bedeutet, und

e) $R^4$ und $R^5$ zusammen mit den sie tragenden Atomen ein bi- oder tricyclisches heterocyclisches Ringsystem mit 5 bis 15 Ring-C-Atomen bilden;
sowie deren physiologisch verträgliche Salze mit Säuren und Basen.

Besonders bevorzugt sind Verbindungen der Formel I, in welchen
n = 1 oder 2 ist
R = Wasserstoff,
Alkyl mit 1- 8 C-Atomen,
Alkenyl mit 2 - 6 C-Atomen,
Cycloalkyl mit 3 - 9 C-Atomen,
Aryl mit 6 - 12 C-Atomen, das durch $(C_1-C_4)$-Alkyl, $(C_1-C_4)$-Alkoxy, Hydroxy, Halogen, Nitro, Amino, Aminoethyl, $(C_1-C_4)$-Alkylamino,Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. biscyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-$(C_1-C_4)$-alkyl,
$(C_1-C_4)$-Alkanoylamino-$(C_1-C_4)$alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4)$-alkylthio,
das im Arylteil wie oben bei Aryl beschrieben substituiert sein kann,
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_6-C_{12})$-Aryloxy-$(C_1-C_4)$-alkyl,
das im Arylteil wie oben bei Aryl beschrieben substituiert sein kann oder
$(C_6-C_{12})$-Aryl-$(C_1-C_4)$-alkoxy,
das im Arylteil wie oben bei Aryl beschrieben substituiert sein kann,

$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
$(C_3-C_9)$-Cycloalkyl-$(C_1-C_4)$-alkyl,

($C_5$-$C_9$)-Cycloalkenyl-($C_1$-$C_4$)-alkyl,
gegebenenfalls teilhydriertes Aryl mit 6 - 12 C-Atomen,
das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürlichen vorkommenden α-Aminosäure $R^1$-CH($NH_2$)-COOH bedeuten
$R^2$ und $R^3$ gleich oder verschieden sind und
Wasserstoff,
Alkyl mit 1- 6 C-Atomen,
Alkenyl mit 2 - 6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6 - 12 C-Atomen,
($C_6$-$C_{12}$)-Aryl-($C_1$-$C_4$)-alkyl,
($C_3$-$C_9$)-Cycloalkyl,
($C_3$-$C_9$)-Cycloalkyl-($C_1$-$C_4$)-alkyl,
Phthalidyl oder
einen Rest der Formel

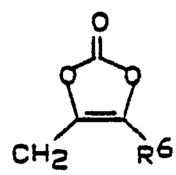

bedeuten, worin $R^6$ Wasserstoff, ($C_1$-$C_6$)-Alkyl oder Aryl mit 6 - 12 C-Atomen ist und wobei mindestens einer der Reste $R^2$ oder $R^3$ einen Rest der Formel

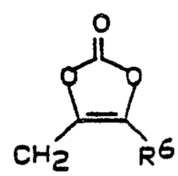

bedeutet, und
$R^4$ und $R^5$ die oben angegebene Bedeutung haben sowie deren physiologisch verträgliche Salze mit Säuren und Basen.

Ganz besonders bevorzugt sind Verbindungen der Formel I, in welchen
n = 1 oder 2 ist,
R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di-oder trisubstituiert sein kann, oder 3- Indolyl, insbesondere Methyl, Ethyl, Cyclohexyl, tert. Butoxycarbonylamino-($C_1$-$C_4$)-alkyl, Benzyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono- oder disubstituiert oder im Falle von Methoxy, trisubstituiert sein kann, bedeutet,

$R^1$ Wasserstoff oder ($C_1$-$C_6$)-Alkyl, das gegebenenfalls durch Amino, ($C_1$-$C_6$)-Acylamino oder Benzoylamino

substituiert sein kann, ($C_2$-$C_6$)-Alkenyl, ($C_3$-$C_9$)- Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_2$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten $\alpha$-Aminosäure, insbesondere aber Wasserstoff, ($C_1$-$C_3$)-Alkyl ($C_2$ oder $C_3$)-Alkenyl, die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl, ($C_6$-$C_{12}$)-Aryl-($C_1$-$C_4$)-alkyl, ($C_1$-$C_5$)-Alkanoyloxy-($C_1$-$C_4$)-alkyl, ($C_1$-$C_6$)-Alkoxycarbonyloxy-($C_1$-$C_4$)-alkyl, ($C_7$-$C_{13}$)-Aroyloxy-($C_1$-$C_4$)-alkyl, ($C_6$-$C_{12}$)-Aryloxycarbonyloxy-($C_1$-$C_4$)-alkyl, Phthalidyl oder einen Rest der Formel

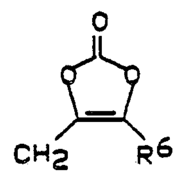

bedeuten, worin $R^6$ Wasserstoff, ($C_1$-$C_6$)-Alkyl oder Aryl mit 6 -12 C-Atomen ist, wobei mindestens einer der Reste $R^2$ oder $R^3$ einen Rest der Formel

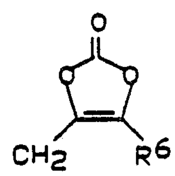

bedeutet,

insbesondere aber Wasserstoff, ($C_1$-$C_4$)-Alkyl, Benzyl, Acetoxymethyl, Propionyloxymethyl, Isopropionlyoxymethyl, n-Butyryloxymethyl, Pivaloyloxymethyl, Isovaleroyloxymethyl, 1-Acetoxyethyl, 1-(n-Propionyloxy)-ethyl, 1-Acetoxypropyl, 1-(Methoxycarbonyloxy)ethyl, 1-(Ethoxycarbonyloxy)ethyl, 1-(Isopropoxycarbonyloxy)-ethyl, 1-(Cyclohexyloxycarbonyloxy)ethyl, Methoxycarbonyloxymethyl, Phthalidyl, (5-Methyl-1,3-dioxolen-2-on-4-yl)methyl, (5-tert. Butyl-1,3-dioxolen-2-on-4-yl)methyl oder (5-Phenyl-1,3-dioxolen-2-on-4-yl)methyl und

$R^4$ und $R^5$ oben angegebene Bedeutung haben sowie deren physiologisch verträgliche Salze mit Säuren und Basen.

Beispiele ganz besonders bevorzugter Verbindungen sind:

2-[N-(1S-Ethoxycarbonyl-3-phenylpropyl)-S-alanyl]-(1S,3S,5S)-2-azabicyclo[3.3.0]octan-3-carbonsäure-(5-methyl-1,3-dioxolen-2-on-4-yl)methylester;

2-[N-(1S-Carboxy-3-phenylpropyl)-S-alanyl]-(1S,3S,5S)-2-azabicyclo[3.3.o]octan-3-carbonsäure-(5-methyl-1,3-dioxolen-2-on-4-yl)methylester;

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]octan-3-carbonsäure-(5-methyl-1,3-dioxolen-2-on-4-yl)methylester;

2-[N-[1S-[(5-Methyl-1,3-dioxolen-2-on-4-yl}methoxycarbonyl]-3-phenylpropyl]-S-alanyl]-(1S,3S,5S)-2-azabicyclo[3.3.0]octan-3-carbonsäurebenzylester;

2-[N-[1S-[(5-Methyl-1,3-dioxolen-2-on-4-yl)-methoxycarbonyl]-3-phenylpropyl]-S-alanyl]-(1S,3S,5S)-2-azabicyclo[3.3.0]octan-3-carbonsäureoctylester;

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]octan-3-carbonsäure;

1-[N-(1S-Ethoxycarbonyl-3-phenylpropyl)-S-alanyl]-(2S,3aR, 7aS)-octahydro[1H]indol-2-carbonsäure-(5-methyl-1,3-dioxolen-2-on-4-yl)methylester;

1-[N-(1S-Carboxy-3-phenylpropyl)-S-alanyl]-(2S,3aR,7aS)-octahydro[1H]indol-2-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäurebenzylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäureoctylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäure;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol--2-carbonsäurebenzhydrylester;

2-[N-(1S-Ethoxycarbonyl-3-phenylpropyl)-S-alanyl]-1,2,3,4-tetrahydroisochinolin-3S-carbonsäure-(5-methyl-1,3-dioxolen-2-on-4-yl)methylester;

2-[N-(1S-Carboxy-3-phenylpropyl)-S-alanyl]-1,2,3,4-tetrahydroisochinolin-3S-carbonsäure-(5-methyl-1,3-dioxolen-2-on-4-yl)methylester;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäure-(5-methyl-1,3-dioxolen-2-on-4-yl)methylester;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäurebenzylester;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäure;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäureoctylester;

1-[N-(1S-Ethoxycarbonyl-3-phenylpropyl)-S-alanyl]-3′S-spiro[(bicyclo[2.2.2]octan)-2,3′-pyrrolidin]-5′S-yl-carbonsäure-(5-methyl-1,3-dioxolen-2-on-4-yl)-methylester;

1-[N-(1S-Carboxy-3-phenylpropyl)-S-alanyl]-3′S-spiro[(biscyclo[2.2.2]octan)-2,3′-pyrrolidin]-5′S-yl-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäurebenzylester;

1-[N-[1S-[(5-Methyl-1,3-dioxolen-2-on-4-yl)methoxycarbonyl]-3-phenylpropyl]-S-alanyl]-3′-spiro-[(bicyclo-[2.2.2]octan)-2,3′-pyrrolidin]-5′S-yl-carbonsäure;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäureoctylester;

1-[N-(1S-Ethoxycarbonyl-butyl)-S-alanyl]-(2S,3aS,7aS)-octahydro[1H]indol-2-carbonsäure-(5-methyl-1,3-dioxolen-2-on-4-yl)methylester;

1-[N-(1S-Carboxy-butyl)-S-alanyl]-(2S,3aS,7aS)-octahydro[1H]indol-2-carbonsäure-(5-methyl-1,3-dioxolen-2-on-4-yl)-methylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäure-(5-methyl-1,3-dioxolen-2-on-4-yl)methylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäurebenzylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäure;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäureoctylester.

Als Salze der Verbindungen der Formel I kommen, je nach saurer oder basischer Natur diese Verbindungen, Alkali-oder Erdalkalisalze oder Salze mit physiologisch verträglichen Aminen oder Salze mit anorganischen oder organischen Säuren wie z. B. HCl, HBr, $H_2SO_4$, Maleinsäure, Fumarsäure, Weinsäure, Citronensäure in Frage.

Die Erfindung betrifft ferner ein Verfahren zur Herstellung von Verbindungen der Formel I, dadurch gekennzeichnet, daß man

a) eine Verbindung der Formel II

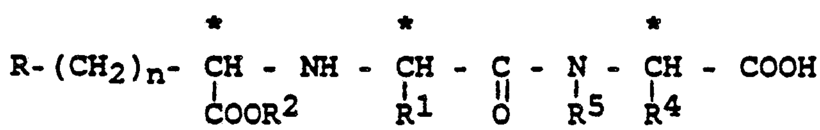

(II)

in der R, $R^1$, $R^2$, $R^4$, $R^5$ und n die gleiche Bedeutung wie in Formel I haben, mit einer Verbindung der Formel III

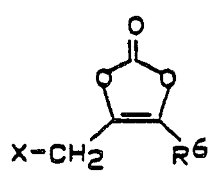

(III)

in der $R^6$ die gleiche Bedeutung wie in Formel I hat und in der X eine Abgangsgruppe, die nucleophil verdrängt werden kann, insbesondere ein Cl, Br, J-Atom oder einen Sulfonsäurerest, vorzugsweise ein Br-Atom bedeutet, unter Bedinungen einer nucleophilen Substitution, vorzugsweise in einem polaren organischen Lösungsmittel, wie einem Alkohol, vorzugsweise Methanol, Ethanol, Propanol oder Isopropanol oder einem niederen Keton, vorzugsweise Aceton, Methylethylketon oder Methylisobutylketon oder in Acetonitril, Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumhydrogencarbonat, Kaliumcarbonat, Natriumhydrogencarbonat, Natriumcarbonat, Triethylamin, Pyridin, 1,8-Diazabicyclo[5,4,0]undec-7-en oder 1,5-Diazabicyclo[4,3,0]non-5-en, sowie mit oder ohne Gegenwart eines Alkalihalogenids, vorzugsweise Natriumjodid oder Kaliumjodid, bei einer Temperatur zwischen -50 und +100° C, vorzugsweise zwischen -20 und +60° C, umsetzt, oder daß man

b) eine Verbindung der Formel IV

$$R-(CH_2)_n-\underset{COOH}{\overset{*}{CH}}-NH-\underset{R^1}{\overset{*}{CH}}-\underset{O}{\overset{\|}{C}}-\underset{R^5}{N}-\underset{R^4}{\overset{*}{CH}}-COOR^3 \qquad (IV)$$

in der R, R', $R^3$, $R^4$, $R^5$ und n die gleiche Bedeutung wie in Formel I haben, mit einer Verbindung der Formel III, wie unter der Verfahrensvariante a) beschrieben, umsetzt, oder daß man,

c) eine Verbindung der Formel V

$$R-(CH_2)_n-\underset{COOR^2}{\overset{*}{CH}}-OSO_2CF_3 \qquad (V)$$

worin R, $R^2$ und n die gleiche Bedeutung wie in Formel I haben, mit einer Verbindung der Formel VI

$$R^3OOC-\underset{R^4}{\overset{*}{CH}}-\underset{R^5}{N}-\underset{O}{\overset{\|}{C}}-\underset{R^1}{\overset{*}{CH}}-NH_2 \qquad (VI)$$

worin R', $R^3$, $R^4$ und $R^5$ die gleiche Bedeutung wie in Formel I haben, z.B. analog der in US-Patent 4525301 beschriebenen Verfahrensweise umsetzt,
oder daß man

d) eine Verbindung der Formel VI mit einer Verbindung der Formel VII

$R^2OOC - CH = CH - CO - R$ (VII)

in welcher R und $R^2$ die gleiche Bedeutung wie in Formel I haben, in bekannter Weise in einer Michael-Reaktion (Organikum, 6. Auflage, S. 492, 1967) umsetzt und die Carbonylgruppe z.B. in sauer alkoholischer Lösung mit einem Edelmetallkatalysator, insbesondere Palladium oder Platin auf Aktivkohle bei einem Druck von 20 bis 120 bar hydriert,
oder daß man

e) eine Verbindung der Formel VI mit einer Verbindung der Formel VIII

$R-(CH_2)_n-\underset{O}{\overset{\|}{C}}-COOR^2$ (VIII)

worin R, $R^2$ und n die gleiche Bedeutung wie in Formel I haben, beispielsweise gemäß der in J. Amer. Chem. Soc. 93, 2897 (1971) beschriebenen Verfahrensweise umsetzt und die erhaltenen Schiff-Basen reduziert, vorzugsweise unter Verwendung eines komplexen Hydrides, z.B. Natriumcyanborhydrid,
und die so erhaltenen Verbindungen der Formel I gegebenenfalls in ihre physiologisch verträglichen Salze überführt.

Verbindungen der Formel II und IV sind bekannt (siehe z.B. EP-A 79022, EP-A 105102, EP-A 113880, EP-A 116270, EP-A 84164, EP-A 90362).

Verbindungen der Formel III sind bekannt, z.B. aus Chem. Pharm. Bull. 32, 2241 (1984), bzw. werden auf analogem Wege aus den entsprechenden Ausgangsmaterialien hergestellt.

Verbindungen der Formel V, in welchen $R^2$ den Rest

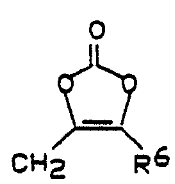

bedeutet, werden aus Verbindungen der Formel IX

$$R\text{-}(CH_2)_n - \underset{\displaystyle COOH}{\underset{|}{CH}} - OH \qquad (IX)$$

worin R und n die gleiche Bedeutung wie in Formel I besitzen, durch Umsetzung mit Verbindungen der Formel III unter den Bedingungen einer nucleophilen Substitution, wie unter der Verfahrensvariante a) beschrieben, und durch anschließende Überführung der Hydroxyfunktion in die $-OSO_2CF_3$-Gruppe nach gängigen Verfahren erhalten.

Verbindungen der Formel VI sind Dipeptide, die aus den einzelnen Aminosäurekomponenten nach an sich bekannten Methoden der Peptidchemie (siehe z.B. Houben-Weyl, Methoden der Organischen Chemie, Bd. XV, Teil II, S. 1-364) aufgebaut werden können.

Verbindungen der Formel VII, in welchen $R^2$ den Rest

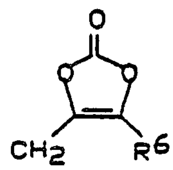

bedeutet, werden aus Verbindungen der Formel X

HOOC - CH = CH - CO - R (X)

in welcher R die gleiche Bedeutung wie in Formel I hat, durch Umsetzung mit Verbindungen der Formel III unter den Bedingungen einer nucleophilen Substitution, wie unter der Verfahrensvariante a) beschrieben, erhalten.

Verbindungen der Formel VIII, in welchen $R^2$ den Rest

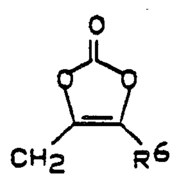

bedeutet, werden aus Verbindungen der Formel XI

$$R\text{-}(CH_2)_n - \underset{\displaystyle O}{\underset{\|}{C}} - COOH \qquad (XI)$$

worin R und n die gleiche Bedeutung wie in Formel I besitzen, durch Umsetzung mit Verbindungen der Formel III unter den Bedingungen einer nucleophilen Substitution, wie unter der Verfahrensvariante a) beschrieben, erhalten.

Verbindungen der Formeln IX, X und XI sind bekannt.

Die Erfindung betrifft ferner neue Zwischenprodukte der Formel V

$$R\text{-}(CH_2)_n - \underset{\displaystyle COOR^2}{\underset{|}{\overset{*}{CH}}} - OSO_2CF_3 \qquad (V)$$

worin R und n gleiche Bedeutung wie in Formel I besitzen und worin $R^2$ einen Rest der Formel

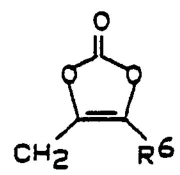

worin $R^6$ Wasserstoff, ein aliphatischer Rest mit 1-6 C-Atomen oder ein gegebenenfalls substituierter aromatischer Rest mit 6-12 C-Atomen ist, bedeutet.

Die Erfindung betrifft ferner ein Verfahren zur Herstellung von Verbindungen der Formel V, worin R und n die gleiche Bedeutung wie in Formel I besitzen und worin $R^2$ einen Rest der Formel

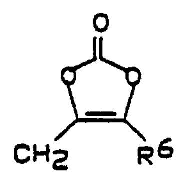

worin $R^6$ Wasserstoff, ein aliphatischer Rest mit 1 - 6 C-Atomen, oder ein gegebenenfalls substituierter aromatischer Rest mit 6 - 12 C-Atomen, bedeutet, dadurch gekennzeichnet, daß man Verbindungen der Formel IX

$$R\text{-}(CH_2)_n - \underset{COOH}{\underset{|}{CH}} - OH \qquad (IX)$$

worin R und n die gleiche Bedeutung wie in Formel I besitzen, mit Verbindungen der Formel III unter den Bedingungen einer nucleophilen Substitution, wie unter der Verfahrensvariante a) beschrieben, umsetzt, und anschließend die Hydroxygruppe in die $-OSO_2CF_3$-Gruppe nach gängigen Verfahren überführt.

Die Verbindungen der Formel I sind Prodrugformen von Inhibitoren des Angiotensin-Converting-Enzyms (ACE-Hemmer). Sie besitzen gegenüber bekannten ACE-Hemmern den Vorteil einer erhöhten Resorbierbarkeit und somit einer erhöhten Bioverfügbarkeit. Unter physiologischen Bedingungen werden sie rasch in die ACE-Hemmer gespalten.

Die Verbindungen der Formel I und deren Salze besitzen lang andauernde, intensive blutdrucksenkende Wirkung. Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-100 mg, vorzugsweise bei 1-40 mg je Einzeldosis bei einem normalgewichtigen erwachsenen Patienten: dies entspricht etwa 15-1300 µg/kg/Tag, vorzugsweise 15-500 µg/kg/Tag. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen der Formel I sind aufgrund ihrer pharmakologischen Eigenschaften neben der Behandlung des Bluthochdruckes auch für die Behandlung cognitiver Dysfunktionen unterschiedlicher Genese, wie sie z.B. bei der Alzheimer'schen Krankheit oder der senilen Demenz auftreten, geeignet. Die nootrope Wirkung der erfindungsgemäßen Verbindungen wurde an Mäusen, die ein Körpergewicht von 20 - 25 besaßen, im inhibitory (passive) avoidance test (step-through-Modell) geprüft. Eine modifizierte Form der von J. KOPP, Z. BODANECKY und M.E. JARVIK beschriebenen Testmethode wurde von J. BURES, O. BURESOVA und J. HUSTON in "Techniques and Basic Experiments for the Study of Brain and Behavior", Elsevier Scientific Publishers, Amsterdam (1983) beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie oder die mittels Scopolamin induzierte Amnesie aufzuheben vermag.

Die Versuche wurden nach mofizierten Testmethoden durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß die Scopolamin-induzierte Amnesie im inhibitory avoidance-Test sich mit einer MED (minimal effective Dosis) von 0,03 - 30 mg/kg p.o. aufheben läßt. Die Vergleichssubstanz hat eine MED von ca. 500-1000 mg/kg p.o.

Die Erfindung betrifft daher weiter die Anwendung der erfindungsgemäßen Verbindungen bei der Behandlung und Prophylaxe cognitiver Dysfunktionen.

Die Erfindung umfaßt weiterhin die genannten Verbindungen enthaltende Arzneimittel, Verfahren zu deren Herstellung sowie die Verwendung der erfindungsgemäßen Verbindungen bei der Herstellung von Arzneimitteln, die zur Behandlung und Prophylaxe der vorstehend genannten Krankheiten eingesetzt werden.

In Ausübung der erfindungsgemäßen Methode können die oben beschreibenen Angiotensin-Converting-Enzyme-Inhibitoren an Säugern wie Affen, Hunden, Katzen, Ratten, Menschen etc angewendet werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Welche Hilfsstoffe für die gewünschte Arzneimittelformulierung geeignet sind, ist dem Fachmann aufgrund seines Fachwissens galäufig. Neben Lösungsmitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral, rektal oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose-oder Mannitlösungen oder auch eine Mischung aus den verschiedenen genannten Lösungsmitteln.

Die nachfolgenden Beispiele sollen die erfindungsgemäßen Verbindungen und Verfahren erläutern, ohne die Erfindung auf die hier stellvertretend genannten Substanzen zu beschränken.

**Beispiel 1**

2-[N-(1S-Ethoxycarbonyl-3-phenylpropyl)-S-alanyl]-(1S,3S,5S)-2-azabiscyclo[3.3.0]octan-3-carbonsäure-(5-methyl-1,3-dioxolen-2-on-4-yl)methylester;

Zu einer Lösung von 2,82 g (7,66 mmol) 2-[N-(1S-Ethoxycarbonyl-3-phenylpropyl)-S-alanyl]-(1S,3S,5S)-2-azabicyclo[3.3.0]octan-3-carbonsäure (Ramipril) in 25 ml absolutem Dimethylformamid werden 1,36 g (13,6 mmol) Kaliumhydrogencarbonat gegeben und 45 Minuten bei 50° C gerührt. Anschließend wird unter Eiskühlung bei 0° C eine Lösung von 1,57 g (8,13 mmol) 4-Brommethyl-5-methyl-1,3-dioxolen-2-on in 7 ml absolutem Dimethylformamid zugetropft, und eine Stunde bei 0° C nachgerührt.

Die Reaktionslösung wird auf 300 ml Wasser gegeben und zweimal mit Essigester extrahiert, die vereinigten Extrakte werden zweimal mit gesättigter Natriumhydrogencarbonatlösung und dreimal mit Wasser gewaschen, über Magnesiumsulfat getrocknet, eingeengt und das Rohprodukt durch Säulenchromatographie an 150 g Kieselgel (Laufmittel Methylenchlorid/Essigester 8:2, 7:3, 1:1) von Verunreinigungen befreit. Man erhält 3,10 g (87 %) farbloses Öl.

$[\alpha]_D^{23}$ = -32,9° (C = 1, Methanol).

Unter Verwendung geeigneter Ausgangsmaterialien können die nachstehenden Verbindungen analog der in Beispiel 1 angegebenen Vorschrift hergestellt werden:

2-[N-(1S-Carboxy-3-phenylpropyl)-S-alanyl]-1,2,3,4-tetrahydroisochinolin-3S-carbonsäure-(5-methyl-1,3-dioxolen-2-on-4-yl)methylester;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäure-(5-methyl-1,3-dioxolen-2-on-4-yl)methylester;

2-[N-[1S-[(5-Methyl-1,3-dioxolen-2-on-4-yl)methoxycarbonyl]- 3-phenylpropyl]-S-alanyl]-1,2,3,4-tetrahydroisochinolin-3S-carbonsäure;

1-[N-(1S-Ethoxycarbonyl-3-phenylpropyl)-S-alanyl]-3′S-spiro[(bicyclo[2.2.2]octan)-2,3′-pyrrolidin]-5′S-yl-carbonsäure-(5-methyl-1,3-dioxolen-2-on-4-yl)-methylester;

1-[N-(1S-Carboxy-3-phenylpropyl)-S-alanyl]-3′S-spiro[(bicyclo[2.2.2]octan)-2,3′-pyrrolidin]-5′S-yl-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäure;

2-[N-(1S-Ethoxycarbonyl-3-phenylpropyl)-S-alanyl]-1,2,3,4-tetrahydroisochinolin-3S-carbonsäure-(5-methyl-1,2-dioxolen-2-on-4-yl)methylester;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäure;

1-[N-(1S-Ethoxycarbonyl-butyl)-S-alanyl]-(2S,3aS,7aS)-octahydro[1H]indol-2-carbonsäure-(5-methyl-1,3-dioxolen-2-on-4-yl)methylester;

1-[N-(1S-Carboxy-butyl)-S-alanyl]-(2S,3aS,7aS)-octahydro-[1H]indol-2-carbonsäure-(5-methyl-1,3-dioxolen-2-on-4-yl)- methylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäure-(5-methyl-1,3-dioxolen-2-on-4-yl)methylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäure;

2-[N-(1S-Carboxy-3-phenylpropyl)-S-alanyl]-(1S,3S,5S)-2-azabicyclo[3.3.0]octan-3-carbonsäure-(5-methyl-1,3-dioxolen-2-on-4-yl)methylester;

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]-octan-3-carbonsäure-(5-methyl-1,3-dioxolan-2-on-4-yl)methylester;

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]-octan-3-carbonsäure;

1-[N-(1S-Ethoxycarbonyl-3-phenylpropyl)-S-alanyl]-(2S,3aR, 7aS)-octahydro[1H]indol-2-carbonsäure-(5-methyl-1,3-dioxolan-2-on-4-yl)methylester;

1-[N-(1S-Carboxy-3-phenylpropyl)-S-alanyl]-(2S,3aR,7aS)-octahydro[1H]indol-2-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäure-(5-methyl-1,3-dioxolen-2-on-4-yl)methylester.

**Beispiel 2**

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]octan-3-carbonsäurebenzylester;

2a)

2R-Hydroxy-4-phenylbuttersäure-(5-methyl-1,3-dioxolen-2-on4-yl)methylester

9,0 g (0,05 mol) 2R-Hydroxy-4-phenylbuttersäure werden in 200 ml absoluten Dimethylformamid gelöst, 10,0 g (0,10 mol) Kaliumhydrogencarbonat zugegeben, 90 Minuten bei 50 °C gerührt und anschließend unter Eiskühlung bei 0 °C eine Lösung von 11,6 g (0,06 mol) 4-Brommethyl-5-methyl-1,3-dioxolen-2-on in 50 ml absolutem Dimethylformamid zugetropft. Man rührt eine Stunde bei 0 °C und 30 Minuten bei Raumtemperatur nach, gibt auf 1 l Wasser und extrahiert zweimal mit Essigester. Die vereinigten Extrakte werden zweimal mit gesättigter Natriumhydrogencarbonatlösung und zweimal mit Wasser gewaschen, über Natriumsulfat getrocknet, eingeengt und das Rohprodukt durch Säulenchromatographie an 600 g Kieselgel (Laufmittel Toluol/Ethanol 199:1) von Verunreinigungen befreit. Man erhält 12,6 g (60 %) farbloses Öl.

$[\alpha]_D^{25}$ = 8,2° (C = 1, Methanol).

2b)

4-Phenyl-2R-trifluormethylsulfonyloxy-buttersäure-(5-methyl-1,3-dioxolen-2-on4-yl)methylester

5,48 g (18,8 mmol) des Alkohols aus a) und 1,6 ml (20 mmol) absolutes Pyridin werden in 100 ml absolutem Methylenchlorid gelöst, auf -10 °C abgekühlt und bei dieser Temperatur innerhalb von 15 Minuten 5,64 g (20 mmol) Trifluormethansulfonsäureanhydrid zugetropft. Es wird noch 10 Minuten bei -10 °C nachgerührt, dann läßt man auf Raumtemperatur kommen, dampft das Lösungsmittel ab, digeriert dan Rückstand in 40 ml Cyclohexan/Essigester 9:1, filtriert von ungelöstem Rückstand ab un reinigt die Produktlösung durch Säulenchromatographie an 200 g Kieselgel (Laufmittel Cyclohexan/Essigester 9:1, 8:2). Man erhält 6,5 g (82 %) farbloses Öl, das sofort weiter umgesetzt wird.

2c)

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]octan-3-carbonsäurebenzylester

4,3 g (10,0 mmol) 2-(S-Alanyl)-(1S,3S,5S)-2-azabicyclo [3.3.0]octan-3-carbonsäurebenzylestertrifluoracetat werden in 25 ml absolutem Methylenchlorid mit 2,7 ml (20,0 mmol) absolutem Triethylamin versetzt, auf 0 °C abgekühlt und bei dieser Temperatur innerhalb von 30 Minuten eine Lösung von 6,5 g des Triflats aus b) in 50 ml absolutem Methylenchlorid zugetropft. Die Reaktionslösung wird 30 Stunden bei Raumtemperatur gerührt, über Nacht stehengelassen, mit Methylenchlorid verdünnt, dreimal mit Wasser gewaschen, über Magnesiumsulfat getrocknet, eingeengt und das Rohprodukt (7,7 g) durch Säulenchromatographie an 400 g Kieselgel (Laufmittel Cyclohexan/Essigester 7:3, 6:4, 1:1, 4:6 von Verunreinigungen

befreit. Man erhält 2.0 g (34 %) farbloses Öl.

$[\alpha]_D^{25}$ = -39,5° (C = 1, Methanol).

Unter Verwendung geeigneter Ausgangsmaterialen können die nachstehenden Verbindungen analog der in Beispiel 2 angegebenen Vorschrift hergestellt werden:

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]octan-3-carbonsäure-(5-methyl-1,3-dioxolen-2-on-4-yl)methylester;

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]octan-3-carbonsäureoctylester;

2-[N-[1S-[(5-Methyl-1,3-dioxolen-2-on-4-yl)methoxycarbonyl]-3-phenylpropyl]-S-alanyl]-(1S,3S,5S)-2-azabicyclo[3.3.0]octan-3-carbonsäure;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäure-(5-methyl-1,3-dioxolen-2-on-4-yl)methylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäurebenzylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-indol-2-carbonsäureoctylester;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäure;

1-[N-[1S-[(5-Methyl-1,3-dioxolen-2-on-4-yl)methoxycarbonyl]-3-phenylpropyl]-S-alanyl]-(2S,3aR,7aS)-octahydro[1H]indol-2-carbonsäurebenzhydrylester;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäure-(5-methyl-1,3-dioxolen-2-on-4-yl)methylester;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäurebenzylester;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäure;

2-[N-[1S-[(5-Methyl-1,3-dioxolen-2-on-4-yl)methoxycarbonyl]-3-phenylpropyl]-S-alanyl]-1,2,3,4-tetrahydroisochinolin-3S-carbonsäureoctylester;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäure-(5-methyl-1,3-dioxolen-2-on-4-yl)methylester;

1-[N-[1S-[(5-Metyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäurebenzylester;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäure;

1-[N-[1S-[(5-Methyl-1,3-dioxolen-2-on-4-yl)methoxycarbonyl]-3-phenylpropyl]-S-alanyl]-3′S-spiro-[(bicyclo-[2.2.2]octan)-2,3′-pyrrolidin]-5′S-yl-carbonsäureoctylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäure-(5-methyl-1,3-dioxolen-2-on-4-yl)methylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäurebenzylester;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäure;

1-[N-[1S-[(5-Methyl-1,3-dioxolen-2-on-4-yl)methoxycarbonyl]-butyl]-S-alanyl]-(2S,3aS,7aS)-octahydro[1H]-indol-2-carbonsäureoctylester.

## Ansprüche

1. Verbindung der Formel I

$$R^3OOC - \underset{R^4}{\overset{*}{CH}} - \underset{R^5}{N} - \underset{O}{\overset{}{C}} - \underset{R^1}{\overset{*}{CH}} - NH - \underset{COOR_2}{\overset{*}{CH}} - (CH_2)_n\text{-}R \qquad (I)$$

in welcher
n = 1 oder 2 ist,
R = Wasserstoff,
einen gegebenenfalls substituierten aliphatischen Rest mit 1 - 21 C-Atomen,
einen gegebenenfalls substituierten alicyclishen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicylischen Rest mit 3 - 20 C-Atomen,
einen gegebenenfalls substituierten alicyclish-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,

$R^2$ und $R^3$ gleich oder verschieden sind und Wasserstoff,
einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen,
einen gegebenenfalls substituierten araliphatischen Rest mit 7 - 32 C-Atomen,
einen Rest der Formel

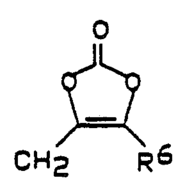

worin $R^5$ Wasserstoff, ein aliphatischer Rest mit 1 - 6 C-Atomen oder ein gegebenenfalls substituierter aromatischer Rest mit 6 -12 C-Atomen ist,
bedeuten,
wobei mindestens einer der Reste $R^2$ oder $R^3$ einen Rest der Formel

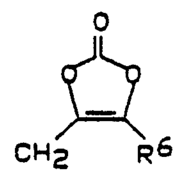

bedeutet und
$R^4$ und $R^5$ zusammen mit den sie tragenden Atomen ein bi- oder tricyclisches heterocyclisches Ringsystem mit 5 bis 15 Ring C-Atomen bilden vorzugsweise aus der Gruppe Tetrahydroisochinolin (A); Decahydroisochinolin (B); Octahydroindol (C); Octahydrocyclopenta[b]pyrrol (D); 2-Azaspiro[4.5]decan (E); 2-Azaspiro-[4.4]-nonan (F); Spiro [(bicyclo[2.2.1]heptan)-2,3′-pyrrolidin] (G); Spiro[(bicyclo[2.2.2]octan)-2,3′-pyrrolidin[ (H); 2-Azatricyclo[$4.3.0.1^{6,9}$]decan (I); Decahydrocyclohepta[b]pyrrol (J);
Hexahydrocyclopropa[b]pyrrol (K); Octahydroisoindol (L); Octahydrocyclopenta-[c]pyrrol (M) 2,3,3a,4, 5,7a-Hexahydroindol (N); 1,2,3,3a,4,6a-Hexahydrocyclopenta[b]pyrrol (O); die alle gegebenenfalls substituiert sein können,
sowie deren physiologisch verträgliche Salze mit Säuren und Basen.

2. Verbindung der Formel I gemäß Anspruch 1, in welcher
a) n = 1 oder 2 ist
b) R 1. Wasserstoff bedeutet,
2. Alkyl mit 1 - 18 C-Atomen bedeutet,
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl, 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;
5. Aryl mit 6 - 12 C-Atomen bedeutet, das durch $(C_1-C_8)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann;
6. falls n = 2 ist, $(C_6-C_{12})$-Aryl-$(C_1-C_8)$-alkyl oder Di-$(C_6-C_{12})$-aryl-$(C_1-C_8)$-alkyl bedeutet, die im Arylteil jeweils wie unter b) 5. beschrieben substituiert sein können; oder
7. Alkoxy mit 1 - 4 C-Atomen;
8. Aryloxy mit 6 - 12 C-Atomen, das wie unter b) 5. beschrieben substituiert sein kann;
9. mono- bzw. bicyclisches Heteroaryloxy oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b) 5. beschrieben substituiert sein können.
10. Amino-$(C_1-C_8)$-alkyl;
11. $(C_1-C_4)$-Alkanoylamino-$(C_1-C_8)$-alkyl;
12. $(C_7-C_{13})$-Aroylamino-$(C_1-C_8)$-alkyl;
13. $(C_1-C_4)$-Alkoxy-carbonylamino-$(C_1-C_8)$-alkyl;
14. $(C_6-C_{12})$-Aryl-$(C_1-C_4)$-alkoxycarbonylamino-$(C_1-C_8)$-alkyl;
15. $(C_6-C_{12})$-Aryl-$(C_1-C_4)$-alkylamino-$(C_1-C_8)$-alkyl;
16. $(C_1-C_4)$-Alkylamino-$(C_1-C_8)$-alkyl;
17. Di-$(C_1-C_4)$-alkylamino-$(C_1-C_8)$-alkyl;
18. Guanidino-$(C_1-C_8)$-alkyl;
19. Imidazolyl;
20. Indolyl;
21. $(C_1-C_4)$-Alkylthio;
22. falls n = 2 ist, $(C_1-C_4)$-Alkylthio-$(C_1-C_8)$-alkyl;
23. $(C_6-C_{12})$-Arylthio-$(C_1-C_8)$-alkyl; das im Arylteil wie unter b) 5. beschrieben, substituiert sein kann,
24. $(C_6-C_{12})$-Aryl-$(C_1-C_8)$-alkylthio, das im Arylteil wie unter b) 5. beschrieben substituiert sein kann;
25. falls n = 2 ist, Carboxy-$(C_1-C_8)$-alkyl;

26. Carboxy;
27. Carbamoyl;
28. falls n = 2 ist, Carbamoyl-($C_1$-$C_8$)-alkyl;
29. ($C_1$-$C_4$)-Alkoxy-carbonyl-($C_1$-$C_8$)-alkyl;
30. falls n = 2 ist, ($C_6$-$C_{12}$)-Aryloxy-($C_1$-$C_8$)-alkyl, das im Arylteil unter b) 5. beschrieben substituiert sein kann; oder
31. ($C_6$-$C_{12}$)-Aryl-($C_1$-$C_8$)-alkoxy,
das im Arylteil unter b) 5. beschrieben substituiert sein kann, bedeutet;

c) $R^1$ 1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Aryl mit 6 - 12 C-Atomen,
das wie unter b) 5. beschrieben substituiert sein kann;
6. ($C_6$-$C_{12}$)-Aryl-($C_1$-$C_8$)-alkyl oder ($C_7$-$C_{13}$)-Aroyl-($C_1$-$C_8$)-alkyl,
die beide wie Aryl unter b) 5. beschrieben substituiert sein können;
7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter b) 5. beschrieben substituiert sein können; oder
8. falls von c) 1. -7. noch nicht umfaßt, die gegebenenfalls geschützte Seitenkette einer natürlich vorkommenden α-Aminosäure der Formel $R^1$-CH($NH_2$)-COOH bedeuten;

d) $R^2$ und $R^3$ gleich oder verschieden sind und
1. Wasserstoff bedeutet;
2. Alkyl mit 1- 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Di-($C_1$-$C_4$)-alkylamino-($C_1$-$C_8$)-alkyl;
6. ($C_1$-$C_5$)-Alkanoyloxy-($C_1$-$C_8$)-alkyl;
7. ($C_1$-$C_6$)-Alkoxy-carbonyloxy-($C_1$-$C_8$)-alkyl;
8. ($C_7$-$C_{13}$)-Aroyloxy-($C_1$-$C_8$)-alkyl;
9. ($C_6$-$C_{12}$)-Aryloxycarbonyloxy($C_1$-$C_8$)-alkyl;
10. Aryl mit 6 - 12 C-Atomen;
11. ($C_7$-$C_{20}$)-Aralkyl;
12. Phthalidyl; oder
13. einen Rest der Formel

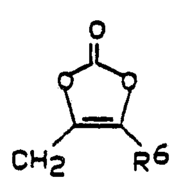

bedeuten, worin $R^6$ Wasserstoff, ($C_1$-$C_6$)-Alkyl oder Aryl mit 6 - 12 C-Atomen ist,
wobei die unter d) 8., 9., 10., 11. und 12. genannten Reste wie unter b) 5. beschrieben im Arylteil substituiert sein können; und
wobei mindestens einer der Reste $R^2$ oder $R^3$ einen Rest der Formel

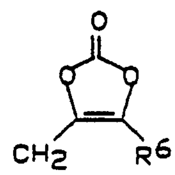

bedeutet, und

e) $R^4$ und $R^5$ zusammen mit den sie tragenden Atomen ein bi - oder tricyclisches heterocyclisches Ringsystem mit 5 bis 15 Ring-C-Atomen bilden;

sowie deren physiologisch verträgliche Salze mit Säuren und Basen.

3. Verbindung der Formel I gemäß Anspruch 1 oder 2, in welcher

n = 1 oder 2 ist

R = Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino,Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,

$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,

$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6 - 12 C-Atomen, das wie oben bei R beschrieben substituiert sein kann, $(C_6-C_{12})$-Aryl-$(C_1-C_4)$-alkyl oder $(C_7-C_{13})$-Aroyl-($C_1$ oder $C_2$)-alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder
1 bis 4 Ringatome Stickstoffatome darstellen,
das wie das vorstehende Aryl substituiert sein kann oder
die gegebenenfalls geschützte Seitenkette einer natürlichen vorkommenden α-Aminosäure $R^1$-$CH(NH_2)$-COOH bedeuten,
$R^2$ und $R^3$ gleich oder verschieden sind und Wasserstoff,
Alkyl mit 1 - 6 C-Atomen,
Alkenyl mit 2 - 6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl,
Aryl mit 6 - 12 C-Atomen,
$(C_6-C_{12})$-Aryl-$(C_1-C_4)$-alkyl,
$(C_3-C_9)$-Cycloalkyl,
$(C_3-C_9)$-Cycloalkyl-$(C_1-C_4)$-alkyl,
Phthalidyl oder
einen Rest der Formel

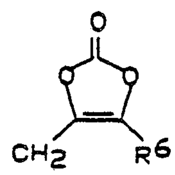

bedeuten, worin $R^6$ Wasserstoff, $(C_1-C_6)$-Alkyl oder Aryl mit 6 - 12 C-Atomen ist und wobei mindestens einer der Reste $R^2$ oder $R^3$ einen Rest der Formel

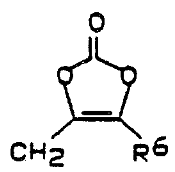

bedeutet, und
$R^4$ und $R^5$ die oben angegebene Bedeutung haben sowie deren physiologisch verträgliche Salze mit Säuren und Basen.

4. Verbindung der Formel I gemäß einem oder mehrerer der Ansprüche 1 bis 3, in welcher
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxy-carbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino-$(C_1-C_4)$-alkyl, Benzyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,

$R^1$ Wasserstoff oder $(C_1-C_6)$-Alkyl, das gegebenenfalls durch Amino, $(C_1-C_6)$-Acylamino oder Benzoylamino

substituiert sein kann, ($C_2$-$C_6$)-Alkenyl, ($C_3$-$C_9$)- Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl, ($C_6$-$C_{12}$)-Aryl-($C_1$-$C_4$)-alkyl, ($C_1$-$C_5$)-Alkanoyloxy-($C_1$-$C_4$)-alkyl, ($C_1$-$C_6$)-Alkoxycarbonyloxy-($C_1$-$C_4$)-alkyl, ($C_7$-$C_{13}$)-Aroyloxy-($C_1$-$C_4$)-alkyl, ($C_6$-$C_{12}$)-Aryloxycarbonyloxy-($C_1$-$C_4$)alkyl, Phthalidyl oder einen Rest der Formel

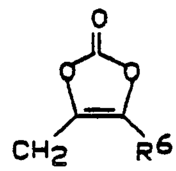

bedeuten,
worin $R^6$ Wasserstoff, ($C_1$-$C_6$)-Alkyl oder Aryl mit 6 - 12 C-Atomen ist, wobei mindestens einer der Reste $R^2$ oder $R^3$ einen Rest der Formel

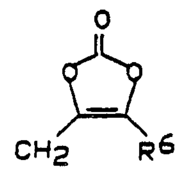

bedeutet,

insbesondere aber Wasserstoff, ($C_1$-$C_4$)-Alkyl, Benzyl, Acetoxymethyl, Propionyloxymethyl, Isopropionyloxymethyl, n-Butyryloxymethyl, Pivaloyloxymethyl, Isovaleroyloxymethyl, 1-Acetoxyethyl, 1-(n-Propionyloxy)-ethyl, 1-Acetoxypropyl, 1-(Methoxycarbonyloxy)ethyl, 1-(Ethoxycarbonyloxy)ethyl, 1-(Isopropoxycarbonyloxy)-ethyl, 1-(Cyclohexyloxycarbonyloxy)ethyl, Methoxycarbonyloxymethyl, Phthalidyl, (5-Methyl-1,3-dioxolen-2-on-4-yl)methyl, (5-tert. Butyl-1,3-dioxolen-2-on-4-yl)methyl oder (5-Phenyl-1,3-dioxolen-2-on-4-yl)methyl und

$R^4$ und $R^5$ die oben angegebene Bedeutung haben, sowie deren physiologisch verträgliche Salze mit Säuren und Basen.

5. Verfahren zur Herstellung einer Verbindung der Formel I gemäß einem der Ansprüche 1 bis 4, dadurch gekennzeichnet, daß man
a) eine Verbindung der Formel II

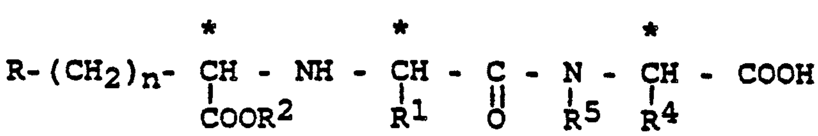 (II)

in der R, $R^1$, $R^2$, $R^4$, $R^5$ und n die gleiche Bedeutung wie in Formel I haben, mit einer Verbindung der Formel III

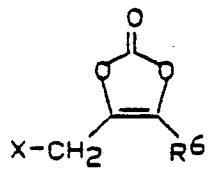

(III)

in der $R^6$ die gleiche Bedeutung wie in Formel I hat und in der X eine Abgangsgruppe, die nucleophil verdrängt werden kann, insbesondere ein Cl, Br, J-Atom oder einen Sulfonsäurerest, vorzugsweise ein Br-Atom bedeutet, unter Bedingungen einer nucleophilen Substitution umsetzt,
oder daß man
b) eine Verbindung der Formel IV

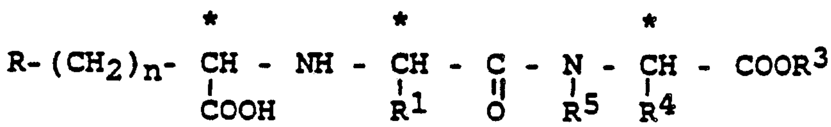

(IV)

in der R, $R^1$, $R^3$, $R^4$, $R^5$ und n die gleiche Bedeutung wie in Formel I haben, mit einer Verbindung der Formel III umsetzt,
oder daß man
c) eine Verbindung der Formel V

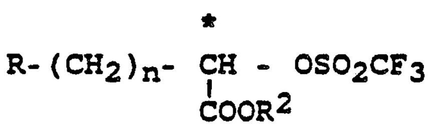

(V)

worin R, $R^2$ und n die gleiche Bedeutung wie in Formel I haben, mit einer Verbindung der Formel VI

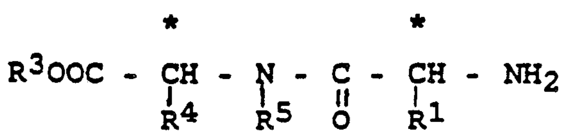

(VI)

worin $R^1$, $R^3$, $R^4$ und $R^5$ die gleiche Bedeutung wie in Formel I haben, umsetzt,
oder daß man
d) eine Verbindung der Formel VI mit einer Verbindung der Formel VII

$R^2OOC - CH = CH - CO - R$ (VII)

in welcher R und $R^2$ die gleiche Bedeutung wie in Formel I haben, umsetzt und die Carbonylgruppe hydriert,
oder daß man
e) eine Verbindung der Formel VI mit einer Verbindung der Formel VIII

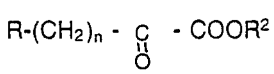

(VIII)

worin R, $R^2$ und n die gleiche Bedeutung wie in Formel I haben, umsetzt und die erhaltenen Schiff-Basen reduziert,
und die so erhaltenen Verbindungen der Formel I gegebenenfalls in ihre physiologisch verträglichen Salze überführt.

6. Verbindung der Formel V

$$R-(CH_2)_n-\underset{COOR^2}{\overset{*}{CH}}-OSO_2CF_3 \qquad (V)$$

worin R und n die gleiche Bedeutung wie in Formel I besitzen und worin $R^2$ einen Rest der Formel

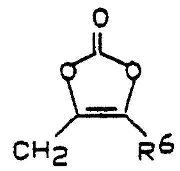

worin $R^6$ Wasserstoff, ein aliphatischer Rest mit 1-6 C-Atomen oder ein gegebenenfalls substituierter aromatischer Rest mit 6-12 C-Atomen ist, bedeutet.

7. Verfahren zur Herstellung einer Verbindung der Formel V gemäß Anspruch 6 worin R und n die gleiche Bedeutung wie in Formel I besitzen und worin $R^2$ einen Rest der Formel

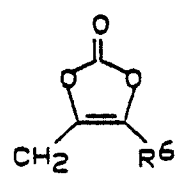

worin $R^6$ Wasserstoff, ein aliphatischer Rest mit 1 - 6 C-Atomen, oder ein gegebenenfalls substituierter aromatischer Rest mit 6 - 12 C-Atomen ist, bedeutet, dadurch gekennzeichnet, daß man eine Verbindung der Formel IX

$$R-(CH_2)_n-\underset{COOH}{CH}-OH \qquad (IX)$$

worin R und n die gleiche Bedeutung wie in Formel I besitzen, mit einer Verbindung der Formel III unter den Bedingungen einer nucleophilen Substitution umsetzt und anschließend die Hydroxygruppe in die $-OSO_2CF_3$-Gruppe überführt.

8. Verbindung gemäß einem oder mehrerer der Ansprüche 1 bis 4 zur Anwendung als Heilmittel.

9. Verbindung gemäß einem oder mehrerer der Ansprüche 1 bis 4 zur Anwendung als Heilmittel bei der Behandlung des Bluthochdrucks.

10. Verbindung gemäß einem oder mehrerer der Ansprüche 1 bis 4 zur Anwendung als Heilmittel bei der Behandlung cognitiver Dysfunktionen.

11. Pharmazeutisches Mittel enthaltend eine Verbindung gemäß einem oder mehrerer der Ansprüche 1 bis 4.

12. Verfahren zur Herstellung eines Mittels gemäß Anspruch 11, dadurch gekennzeichnet, daß man eine Verbindung der Formel I oder deren physiologisch verträgliches Salz und einen physiologisch annehmbaren Träger gegebenenfalls zusammen mit weiteren Zusatz- oder Hilfsstoffen in eine geeignete Darreichungsform bringt.

Patentansprüche für den folgenden Vertragsstaat: GR

1. Verfahren zur Herstellung einer Verbindung der Formel I

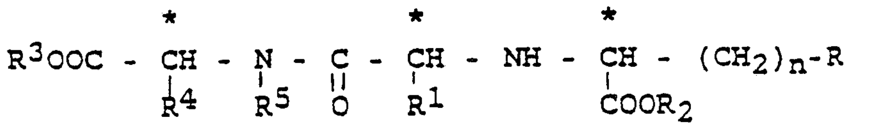

(I)

in welcher
n = 1 oder 2 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen,
oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden $\alpha$-Aminosäure bedeutet,

$R^2$ und $R^3$ gleich oder verschieden sind und Wasserstoff,
einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen,
einen gegebenenfalls substituierten araliphatischen Rest mit 7 - 32 C-Atomen,
einen Rest der Formel

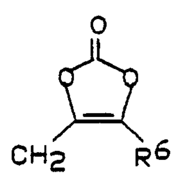

worin $R^6$ Wasserstoff, ein aliphatischer Rest mit 1 - 6 C-Atomen oder ein gegebenenfalls substituierter aromatischer Rest mit 6 -12 C-Atomen ist,
bedeuten,
wobei mindestens einer der Reste $R^2$ oder $R^3$ einen Rest der Formel

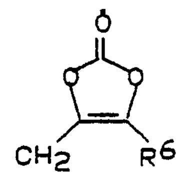

bedeutet und

$R^4$ und $R^5$ zusammen mit den sie tragenden Atomen ein bi- oder tricyclisches heterocyclisches Ringsystem mit 5 bis 15 Ring-C-Atomen bilden vorzugsweise aus der Gruppe Tetrahydroisochinolin (A); Decahydroisochinolin (B); Octahydroindol (C); Octahydrocyclopenta-[b]pyrrol (D); 2-Azaspiro[4.5]decan (E); 2-Azaspiro-[4.4]-nonan (F); Spiro -[(bicyclo[2.2.1]heptan)-2,3′-pyrrolidin] (G); Spiro[(bicyclo[2.2.2]octan)-2,3′-pyrrolidin] (H); 2-Azatricyclo[4.3.0.1$^{6,9}$]decan (I); Decahydrocyclohepta[b]pyrrol (J); Hexahydrocyclopropa[b]pyrrol (K); Octahydroisoindol (L); Octahydrocyclopenta-[c]pyrrol (M) 2,3,3a,4,5,7a-Hexahydroindol (N); 1,2,3,3a,4,6a-Hexahydrocylopenta[b]pyrrol (O); die alle gegebenenfalls substituiert sein können,

sowie deren physiologisch verträglichen Salze mit Säuren und Basen, dadurch gekennzeichnet, daß man

a) eine Verbindung der Formel II

$$R-(CH_2)_n-\overset{*}{C}H(COOR^2)-NH-\overset{*}{C}H(R^1)-C(=O)-N(R^5)-\overset{*}{C}H(R^4)-COOH \qquad (II)$$

in der R, R′, $R^2$, $R^4$, $R^5$ und n die gleiche Bedeutung wie in Formel I haben, mit einer Verbindung der Formel III

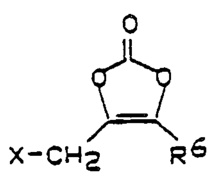

(III)

in der $R^5$ die gleiche Bedeutung wie in Formel I hat und in der X eine Abgangsgruppe, die nucleophil verdrängt werden kann, insbesondere ein Cl, Br, J-Atom oder einen Sulfonsäurerest, vorzugsweise ein Br-Atom bedeutet, unter Bedingungen einer nucleophilen Substitution umsetzt,

oder daß man

b) eine Verbindung der Formel IV

$$R-(CH_2)_n-\overset{*}{C}H(COOH)-NH-\overset{*}{C}H(R^1)-C(=O)-N(R^5)-\overset{*}{C}H(R^4)-COOR^3 \qquad (IV)$$

in der R, R′, $R^3$, $R^4$, $R^5$ und n die gleiche Bedeutung wie in Formel I haben, mit einer Verbindung der Formel III umsetzt,

oder daß man

c) eine Verbindung der Formel V

$$R-(CH_2)_n-\overset{*}{C}H(COOR^2)-OSO_2CF_3 \qquad (V)$$

worin R, $R^2$ und n die gleiche Bedeutung wie in Formel I haben, mit einer Verbindung der Formel VI

$$R^3OOC - \overset{*}{\underset{R^4}{CH}} - \underset{R^5}{N} - \underset{O}{\overset{\parallel}{C}} - \overset{*}{\underset{R^1}{CH}} - NH_2 \qquad (VI)$$

worin $R^1$, $R^3$, $R^4$ und $R^5$ die gleiche Bedeutung wie in Formel I haben, umsetzt,
oder daß man
d) eine Verbindung der Formel VI mit einer Verbindung der Formel VII

$R^2OOC - CH = CH - CO - R$ (VII)

in welcher R und $R^2$ die gleiche Bedeutung wie in Formel I haben, umsetzt und die Carbonylgruppen hydriert,
oder daß man
e) eine Verbindung der Formel VI mit einer Verbindung der Formel VIII

$R-(CH_2)_n - \underset{O}{\overset{}{C}} - COOR^2$ (VIII)
worin R, $R^2$ und n die gleiche Bedeutung wie in Formel I haben, umsetzt und die erhaltenen Schiff-Basen reduziert,
und die so erhaltenen Verbindungen der Formel I gegebenenfalls in ihre physiologisch verträglichen Salze überführt.

2. Verfahren gemäß Anspruch 1, dadurch gekennzeichnet, daß eine Verbindung der Formel I hergestellt wird, in welcher
a) n = 1 oder 2 ist
b) R 1. Wasserstoff bedeutet,
2. Alkyl mit 1 - 18 C-Atomen bedeutet,
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;
5. Aryl mit 6 - 12 C-Atomen bedeutet, das durch $(C_1-C_8)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann;
6. falls n = 2 ist, $(C_6-C_{12})$-Aryl-$(C_1-C_8)$-alkyl oder Di-$(C_6-C_{12})$-aryl-$(C_1-C_8)$-alkyl bedeutet, die im Arylteil jeweils wie unter b) 5. beschrieben substituiert sein können; oder
7. Alkoxy mit 1 - 4 C-Atomen;
8. Aryloxy mit 6 - 12 C-Atomen, das wie unter b) 5. beschrieben substituiert sein kann;
9. mono- bzw. bicyclisches Heteroaryloxy oder Heteroaryl-$(C_1-C_8)$-alkyl mit 5 - 7 bzw. 8 - 10 Ringatomen, wovon bis zu 9 Ringatome Kohlenstoff und 1 bis 12 Ringatome Schwefel oder Sauerstoff und/oder 1 bis 4 Ringatome Stickstoff darstellen, die im Heteroaryl wie unter b) 5. beschrieben substituiert sein können.
10. Amino-$(C_1-C_8)$-alkyl;
11. $(C_1-C_4)$-Alkanoylamino-$(C_1-C_8)$-alkyl;
12. $(C_7-C_{13})$-Aroylamino-$(C_1-C_8)$-alkyl;
13. $(C_1-C_4)$-Alkoxy-carbonylamino-$(C_1-C_8)$-alkyl;
14. $(C_6-C_{12})$-Aryl-$(C_1-C_4)$-alkoxycarbonylamino-$(C_1-C_8)$-alkyl;
15. $(C_6-C_{12})$-Aryl-$(C_1-C_4)$-alkylamino-$(C_1-C_8)$-alkyl;
16. $(C_1-C_4)$-Alkylamino-$(C_1-C_8)$-alkyl;
17. Di-$(C_1-C_4)$-alkylamino-$(C_1-C_8)$-alkyl;
18. Guanidino-$(C_1-C_8)$-alkyl;
19. Imidazolyl;
20. Indolyl;
21. $(C_1-C_4)$-Alkylthio;
22. falls n = 2 ist, $(C_1-C_4)$-Alkylthio-$(C_1-C_8)$-alkyl;
23. $(C_6-C_{12})$-Arylthio-$(C_1-C_8)$-alkyl;
das im Arylteil wie unter b) 5. beschrieben, substituiert sein kann,

24. ($C_6$-$C_{12}$)-Aryl-($C_1$-$C_8$)-alkylthio, das im Arylteil wie unter b) 5. beschrieben substituiert sein kann;
25. falls n = 2 ist, Carboxy-($C_1$-$C_8$)-alkyl;
26. Carboxy;
27. Carbamoyl;
28. falls n = 2 ist, Carbamoyl-($C_1$-$C_8$)-alkyl;
29. ($C_1$-$C_4$)-Alkoxy-carbonyl-($C_1$-$C_8$)-alkyl;
30. falls n = 2 ist, ($C_6$-$C_{12}$)-Aryloxy-($C_1$-$C_8$)-alkyl, das im Arylteil unter b) 5. beschrieben substituiert sein kann; oder
31. ($C_6$-$C_{12}$)-Aryl-($C_1$-$C_8$)-alkoxy, das im Arylteil unter b) 5. beschrieben substituiert sein kann, bedeutet;
c) R' 1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Aryl mit 6 - 12 C-Atomen, das wie unter b) 5. beschrieben substituiert sein kann;
6. ($C_6$-$C_{12}$)-Aryl-($C_1$-$C_8$)-alkyl oder ($C_7$-$C_{13}$)-Aroyl-($C_1$-$C_8$)-alkyl, die beide wie Aryl unter b) 5. beschrieben substituiert sein können;
7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter b) 5. beschrieben substituiert sein können; oder
8. falls von c) 1. -7. noch nicht umfaßt, die gegebenenfalls geschützte Seitenkette einer natürlich vorkommenden α-Aminosäure der Formel $R^1$-CH($NH_2$)-COOH bedeuten;
d) $R^2$ und $R^3$ gleich oder verschieden sind und
1. Wasserstoff bedeutet;
2. Alkyl mit 1 - 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Di-($C_1$-$C_4$)-alkylamino-($C_1$-$C_8$)-alkyl;
6. ($C_1$-$C_5$)-Alkanoyloxy-($C_1$-$C_8$)-alkyl;
7. ($C_1$-$C_6$)-Alkoxy-carbonyloxy-($C_1$-$C_8$)-alkyl;
8. ($C_7$-$C_{13}$)-Aroyloxy-($C_1$-$C_8$)-alkyl;
9. ($C_6$-$C_{12}$)-Aryloxycarbonyloxy($C_1$-$C_8$)-alkyl;
10. Aryl mit 6 - 12 C-Atomen;
11. ($C_7$-$C_{20}$)-Aralkyl;
12. Phthalidyl; oder
13. einen Rest der Formel

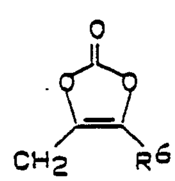

bedeuten, worin $R^6$ Wasserstoff, ($C_1$-$C_6$)-Alkyl oder Aryl mit 6 - 12 C-Atomen ist, wobei die unter d) 8., 9., 10., 11. und 12. genannten Reste wie unter b) 5. beschrieben im Arylteil substituiert sein können; und

wobei mindestens einer der Reste $R^2$ oder $R^3$ einen Rest der Formel

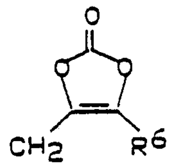

bedeutet, und

e) $R^4$ und $R^5$ zusammen mit den sie tragenden Atomen ein bi- oder tricyclisches heterocyclisches Ringsystem mit 5 bis 15 Ring-C-Atomen bilden;

oder deren physiologisch verträglichen Salze mit Säuren und Basen.

3. Verfahren gemäß Anspruch 1 oder 2, dadurch gekennzeichnet, daß eine Verbindung der Formel I hergestellt wird, in welcher

n = 1 oder 2 ist

R = Wasserstoff,

Alkyl mit 1 - 8 C-Atomen,

Alkenyl mit 2 - 6 C-Atomen,

Cycloalkyl mit 3 - 9 C-Atomen,

Aryl mit 6 - 12 C-Atomen,

das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,

Alkoxy mit 1 - 4 C-Atomen,

Aryloxy mit 6 - 12 C-Atomen,

das wie oben bei Aryl beschrieben substituiert sein kann,

mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,

das wie oben bei Aryl beschrieben substituiert sein kann,

Amino-($C_1$-$C_4$)-alkyl,

($C_1$-$C_4$)-Alkanoylamino-($C_1$-$C_4$)alkyl,

($C_7$-$C_{13}$)-Aroylamino-($C_1$-$C_4$)-alkyl,

($C_1$-$C_4$)-Alkoxy-carbonylamino-($C_1$-$C_4$)-alkyl,

($C_6$-$C_{12}$)-Aryl-($C_1$-$C_4$)-alkoxycarbonylamino-($C_1$-$C_4$)-alkyl,

($C_6$-$C_{12}$)-Aryl-($C_1$-$C_4$)-alkylamino-($C_1$-$C_4$)-alkyl,

($C_1$-$C_4$)-Alkylamino-($C_1$-$C_4$)-alkyl,

Di-($C_1$-$C_4$)-alkylamino-($C_1$-$C_4$)-alkyl,

Guanidino-($C_1$-$C_4$)-alkyl,

Imidazolyl, Indolyl,

($C_1$-$C_4$)-Alkylthio,

($C_1$-$C_4$)-Alkylthio-($C_1$-$C_4$)-alkyl,

($C_6$-$C_{12}$)-Arylthio-($C_1$-$C_4$)-alkyl,

das im Arylteil wie oben bei Aryl beschrieben, substituiert sein kann,

($C_6$-$C_{12}$)-Aryl-($C_1$-$C_4$)-alkylthio,

das im Arylteil wie oben bei Aryl beschrieben substituiert sein kann,

Carboxy-($C_1$-$C_4$)-alkyl,

Carboxy, Carbamoyl,

Carbamoyl-($C_1$-$C_4$)-alkyl,

($C_1$-$C_4$)-Alkoxy-carbonyl-($C_1$-$C_4$)-alkyl,

($C_6$-$C_{12}$)-Aryloxy-($C_1$-$C_4$)-alkyl,

das im Arylteil wie oben bei Aryl beschrieben substituiert sein kann oder

($C_6$-$C_{12}$)-Aryl-($C_1$-$C_4$)-alkoxy,

das im Arylteil wie oben bei Aryl beschrieben substituiert sein kann,

$R^1$ Wasserstoff,

Alkyl mit 1 - 6 C-Atomen,

Alkenyl mit 2 - 6 C-Atomen,

Alkinyl mit 2 - 6 C-Atomen,
Cycloalkyl mit 3 - 9 C-Atomen,
Cycloalkenyl mit 5 - 9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6 - 12 C-Atomen, das wie oben bei R beschrieben substituiert sein kann, $(C_6-C_{12})$-Aryl-$(C_1-C_4)$-alkyl oder $(C_7-C_{13})$-Aroyl-($C_1$ oder $C_2$)-alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder
1 bis 4 Ringatome Stickstoffatome darstellen, das wie das vorstehende Aryl substituiert sein kann oder
die gegebenenfalls geschützte Seitenkette einer natürlichen vorkommenden α-Aminosäure $R^1$-CH($NH_2$)-COOH bedeuten,
$R^2$ und $R^3$ gleich oder verschieden sind und
Wasserstoff,
Alkyl mit 1 - 6 C-Atomen,
Alkenyl mit 2 - 6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl,
Aryl mit 6 - 12 C-Atomen,
$(C_6-C_{12})$-Aryl-$(C_1-C_4)$-alkyl,
$(C_3-C_9)$-Cycloalkyl,
$(C_3-C_9)$-Cycloalkyl-$(C_1-C_4)$-alkyl,
Phthalidyl oder
einen Rest der Formel

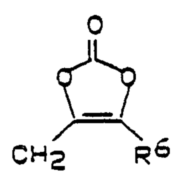

bedeuten, worin $R^6$ Wasserstoff, $(C_1-C_6)$-Alkyl oder Aryl mit 6 - 12 C-Atomen ist und wobei mindestens einer der Reste $R^2$ oder $R^3$ einen Rest der Formel

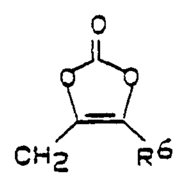

bedeutet, und
$R^4$ und $R^5$ die oben angegebene Bedeutung haben sowie deren physiologisch verträglichen Salze mit Säuren und Basen.

4. Verfahren gemäß einem oder mehreren der Ansprüche 1 bis 3, dadurch gekennzeichnet, daß eine Verbindung der Formel I hergestellt wird, in welcher
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxy-carbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino-$(C_1-C_4)$-alkyl, Benzyloxycarbonylamino-$(C_1-C_4)$-alkyl oder Phenyl, das durch Phenyl, $(C_1-C_2)$-Alkyl, ($C_1$ oder

$C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono- oder disubstituiert oder im Falle von Methoxy, trisubstituiert sein kann, bedeutet,

$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)- Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten $\alpha$-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl, ($C_6$-$C_{12}$)-Aryl-($C_1$-$C_4$)-alkyl, ($C_1$-$C_5$)-Alkanoyloxy-($C_1$-$C_4$)-alkyl, ($C_1$-$C_6$)-Alkoxycarbonyloxy-($C_1$-$C_4$)-alkyl, ($C_7$-$C_{13}$)-Aroyloxy-($C_1$-$C_4$)-alkyl, ($C_6$-$C_{12}$)-Aryloxycarbonyloxy-($C_1$-$C_4$)alkyl, Phthalidyl oder einen Rest der Formel

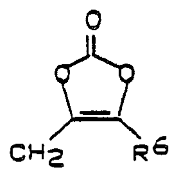

bedeutet,
worin $R^6$ Wasserstoff, ($C_1$-$C_6$)-Alkyl oder Aryl mit 6 - 12 C-Atomen ist, wobei mindestens einer der Reste $R^2$ oder $R^3$ einen Rest der Formel

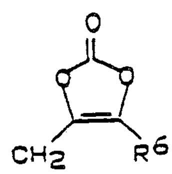

bedeutet,
insbesondere aber Wasserstoff, ($C_1$-$C_4$)-Alkyl, Benzyl, Acetoxymethyl, Propionyloxymethyl, Isopropionyloxymethyl, n-Butyryloxymethyl, Pivaloyloxymethyl, Isovaleroyloxymethyl, 1-Acetoxyethyl, 1-(n-Propionyloxy)-ethyl, 1-Acetoxypropyl, 1-(Methoxycarbonyloxy)ethyl, 1-(Ethoxycarbonyloxy)ethyl, 1-(Isopropoxycarbonyloxy)-ethyl, 1-(Cyclohexyloxycarbonyloxy)ethyl, Methoxycarbonyloxymethyl, Phthalidyl, (5-Methyl-1,3-dioxolen-2-on-4-yl)methyl, (5-tert. Butyl-1,3-dioxolen-2-on-4-yl)methyl oder (5-Phenyl-1,3-dioxolen-2-on-4-yl)methyl und
$R^4$ und $R^5$ die oben angegebene Bedeutung haben, oder deren physiologisch verträglichen Salze mit Säuren und Basen.

5. Verfahren zur Herstellung einer Verbindungen der Formel V

$$R\text{-}(CH_2)_n - \overset{*}{C}H(COOR^2) - OSO_2CF_3 \qquad (V)$$

worin R und n die gleiche Bedeutung wie in Formel I besitzen und worin $R^2$ einen Rest der Formel

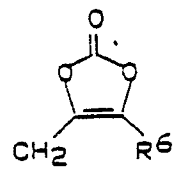

worin $R^6$ Wasserstoff, ein aliphatischer Rest mit 1 - 6 C-Atomen, oder ein gegebenenfalls substituierter aromatischer Rest mit 6 - 12 C-Atomen ist, bedeutet, dadurch gekennzeichnet, daß man eine Verbindung der Formel IX

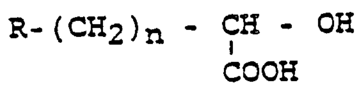
(IX)

worin R und n die gleiche Bedeutung wie in Formel I besitzen, mit einer Verbindung der Formel III unter den Bedingungen einer nucleophilen Substitution umsetzt und anschließend die Hydroxygruppe in die $-OSO_2CF_3$-Gruppe überführt.

6. Verfahren zur Herstellung eines pharmazeutischen Mittels enthaltend eine Verbindung gemäß einem oder mehrerer der Ansprüche 1 bis 4, dadurch gekennzeichnet, daß man eine Verbindung der Formel I oder deren physiologisch verträgliches Salz und einen physiologisch annehmbaren Träger gegebenenfalls zusammen mit weiteren Zusatz- oder Hilfsstoffen in eine geeignete Darreichungsform bringt.

7. Verbindung der Formel V

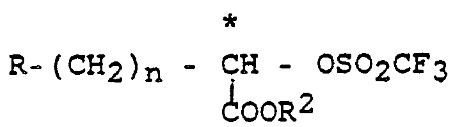
(V)

worin R und n die gleiche Bedeutung wie in Formel I besitzen und worin $R^2$ einen Rest der Formel

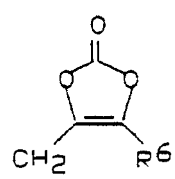

worin $R^6$ Wasserstoff, ein aliphatischer Rest mit 1-6 C-Atomen oder ein gegebenenfalls substituierter aromatischer Rest mit 6-12 C-Atomen ist, bedeutet.

Patentansprüche für den folgenden Vertragsstaat:ES

1. Verfahren zur Herstellung einer Verbindung der Formel I

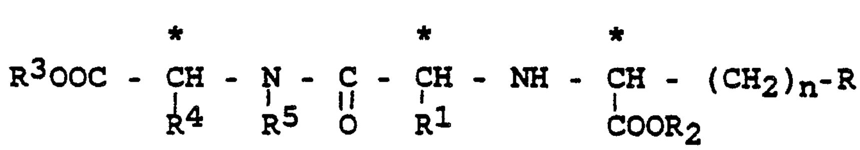
(I)

in welcher
n = 1 oder 2 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,

einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen,
oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,

$R^2$ und $R^3$ gleich oder verschieden sind und Wasserstoff,
einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen,
einen gegebenenfalls substituierten araliphatischen Rest mit 7 - 32 C-Atomen,
einen Rest der Formel

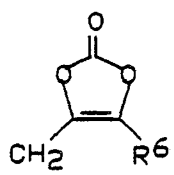

worin $R^6$ Wasserstoff, ein aliphatischer Rest mit 1 - 6 C-Atomen oder ein gegebenenfalls substituierter aromatischer Rest mit 6 -12 C-Atomen ist,
bedeuten,
wobei mindestens einer der Reste $R^2$ oder $R^3$ einen Rest der Formel

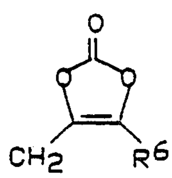

bedeutet und
$R^4$ und $R^5$ zusammen mit den sie tragenden Atomen ein bi- oder tricyclisches heterocyclisches Ringsystem mit 5 bis 15 Ring-C-Atomen bilden vorzugsweise aus der Gruppe Tetrahydroisochinolin (A); Decahydroisochinolin (B); Octahydroindol (C); Octahydrocyclopenta-[b]pyrrol (D); 2-Azaspiro[4.5]decan (E); 2-Azaspiro-[4.4]-nonan (F); Spiro -[(bicyclo[2.2.1]heptan)-2,3′-pyrrolidin] (G); Spiro[(bicyclo[2.2.2]octan)-2,3′-pyrrolidin] (H); 2-Azatricyclo[4.3.0.1$^{6,9}$]decan (I); Decahydrocyclohepta[b]pyrrol (J); Hexahydrocyclopropa[b]pyrrol (K); Octahydroisoindol (L); Octahydrocyclopenta-[c]pyrrol (M) 2,3,3a,4,5,7a-Hexahydroindol (N); 1,2,3,3a,4,6a-Hexahydrocyclopenta[b]pyrrol (O); die alle gegebenenfalls substituiert sein können,
sowie deren physiologisch verträglichen Salze mit Säuren und Basen, dadurch gekennzeichnet, daß man
a) eine Verbindung der Formel II

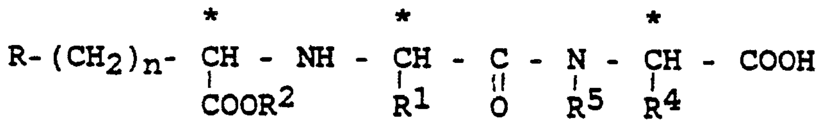 (II)

in der R, R', $R^2$, $R^4$, $R^5$ und n die gleiche Bedeutung wie in Formel I haben, mit einer Verbindung der Formel III

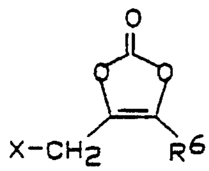 (III)

in der $R^6$ die gleiche Bedeutung wie in Formel I hat und in der X eine Abgangsgruppe, die nucleophil verdrängt werden kann, insbesondere ein Cl, Br, J-Atom oder einen Sulfonsäurerest, vorzugsweise ein Br-Atom bedeutet, unter Bedingungen einer nucleophilen Substitution umsetzt,
oder daß man
b) eine Verbindung der Formel IV

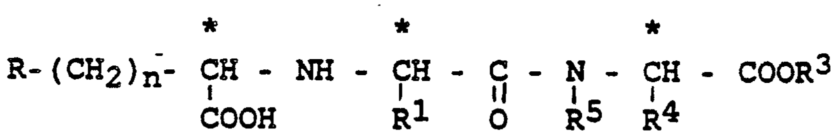 (IV)

in der R, R', $R^3$, $R^4$, $R^5$ und n die gleiche Bedeutung wie in Formel I haben, mit einer Verbindung der Formel III umsetzt,
oder daß man
c) eine Verbindung der Formel V

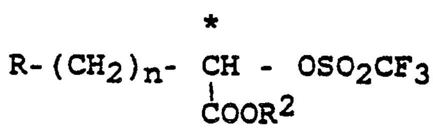 (V)

worin R, $R^2$ und n die gleiche Bedeutung wie in Formel I haben, mit einer Verbindung der Formel VI

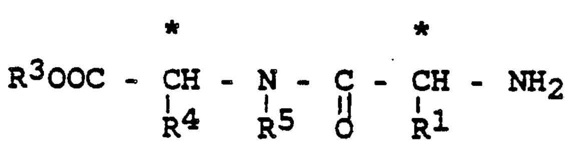 (VI)

worin R', $R^3$, $R^4$ und $R^5$ die gleiche Bedeutung wie in Formel I haben, umsetzt,
oder daß man
d) eine Verbindung der Formel VI mit einer Verbindung der Formel VII

$R^2OOC - CH = CH - CO - R$ (VII)

in welcher R und $R^2$ die gleiche Bedeutung wie in Formel I haben, umsetzt und die Carbonylgruppen hydriert,
oder daß man
e) eine Verbindung der Formel VI mit einer Verbindung der Formel VIII

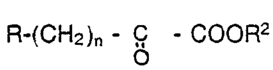 (VIII)

worin R, $R^2$ und n die gleiche Bedeutung wie in Formel I haben, umsetzt und die erhaltenen Schiff-Basen reduziert,
und die so erhaltenen Verbindungen der Formel I gegebenenfalls in ihre physiologisch verträglichen Salze überführt.

2. Verfahren gemäß Anspruch 1, dadurch gekennzeichnet, daß eine Verbindung der Formel I hergestellt wird, in welcher
a) n = 1 oder 2 ist
b) R 1. Wasserstoff bedeutet,
2. Alkyl mit 1 - 18 C-Atomen bedeutet,
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;
5. Aryl mit 6 - 12 C-Atomen bedeutet, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann;
6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter b) 5. beschrieben substituiert sein können; oder
7. Alkoxy mit 1 - 4 C-Atomen;
8. Aryloxy mit 6 - 12 C-Atomen,
das wie unter b) 5. beschrieben substituiert sein kann;
9. mono- bzw. bicyclisches Heteroaryloxy oder Heteroaryl-($C_1$-$C_8$)-alkyl mit 5 - 7 bzw. 8 - 10 Ringatomen, wovon bis zu 9 Ringatome Kohlenstoff und 1 bis 12 Ringatome Schwefel oder Sauerstoff und/oder 1 bis 4 Ringatome Stickstoff darstellen, die im Heteroaryl wie unter b) 5. beschrieben substituiert sein können.
10. Amino-($C_1$-$C_8$)-alkyl;
11. ($C_1$-$C_4$)-Alkanoylamino-($C_1$-$C_8$)-alkyl;
12. ($C_7$-$C_{13}$)-Aroylamino-($C_1$-$C_8$)-alkyl;
13. ($C_1$-$C_4$)-Alkoxy-carbonylamino-($C_1$-$C_8$)-alkyl;
14. ($C_6$-$C_{12}$)-Aryl-($C_1$-$C_4$)-alkoxycarbonylamino-($C_1$-$C_8$)-alkyl;
15. ($C_6$-$C_{12}$)-Aryl-($C_1$-$C_4$)-alkylamino-($C_1$-$C_8$)-alkyl;
16. ($C_1$-$C_4$)-Alkylamino-($C_1$-$C_8$)-alkyl;
17. Di-($C_1$-$C_4$)-alkylamino-($C_1$-$C_8$)-alkyl;
18. Guanidino-($C_1$-$C_8$)-alkyl;
19. Imidazolyl;
20. Indolyl;
21. ($C_1$-$C_4$)-Alkylthio;
22. falls n = 2 ist, ($C_1$-$C_4$)-Alkylthio-($C_1$-$C_8$)-alkyl;
23. ($C_6$-$C_{12}$)-Arylthio-($C_1$-$C_8$)-alkyl;
das im Arylteil wie unter b) 5. beschrieben, substituiert sein kann,
24. ($C_6$-$C_{12}$)-Aryl-($C_1$-$C_8$)-alkylthio,
das im Arylteil wie unter b) 5. beschrieben substituiert sein kann;
25. falls n = 2 ist, Carboxy-($C_1$-$C_8$)-alkyl;
26. Carboxy;
27. Carbamoyl;
28. falls n = 2 ist, Carbamoyl-($C_1$-$C_8$)-alkyl;
29. ($C_1$-$C_4$)-Alkoxy-carbonyl-($C_1$-$C_8$)-alkyl;
30. falls n = 2 ist, ($C_6$-$C_{12}$)-Aryloxy-($C_1$-$C_8$)-alkyl, das im Arylteil unter b) 5. beschrieben substituiert sein kann; oder
31. ($C_6$-$C_{12}$)-Aryl-($C_1$-$C_8$)-alkoxy,
das im Arylteil unter b) 5. beschrieben substituiert sein kann, bedeutet;
c) $R^1$ 1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Aryl mit 6 - 12 C-Atomen,
das wie unter b) 5. beschrieben substituiert sein kann;
6. $(C_6-C_{12})$-Aryl-$(C_1-C_8)$-alkyl oder $(C_7-C_{13})$-Aroyl-$(C_1-C_8)$-alkyl,
die beide wie Aryl unter b) 5. beschrieben substituiert sein können;
7. mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter b) 5. beschrieben substituiert sein können; oder
8. falls von c) 1. -7. noch nicht umfaßt, die gegebenenfalls geschützte Seitenkette einer natürlich vorkommenden α-Aminosäure der Formel $R^1$-CH($NH_2$)-COOH bedeuten;
d) $R^2$ und $R^3$ gleich oder verschieden sind und
1. Wasserstoff bedeutet;
2. Alkyl mit 1 - 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Di-$(C_1-C_4)$-alkylamino-$(C_1-C_8)$-alkyl;
6. $(C_1-C_5)$-Alkanoyloxy-$(C_1-C_8)$-alkyl;
7. $(C_1-C_6)$-Alkoxy-carbonyloxy-$(C_1-C_8)$-alkyl;
8. $(C_7-C_{13})$-Aroyloxy-$(C_1-C_8)$-alkyl;
9. $(C_6-C_{12})$-Aryloxycarbonyloxy$(C_1-C_8)$-alkyl;
10. Aryl mit 6 - 12 C-Atomen;
11. $(C_7-C_{20})$-Aralkyl;
12. Phthalidyl; oder
13. einen Rest der Formel

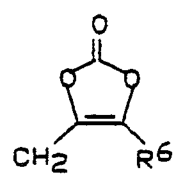

bedeuten, worin $R^6$ Wasserstoff, $(C_1-C_6)$-Alkyl oder Aryl mit 6 - 12 C-Atomen ist,
wobei die unter d) 8., 9., 10., 11. und 12. genannten Reste wie unter b) 5. beschrieben im Arylteil substituiert sein können; und
wobei mindestens einer der Reste $R^2$ oder $R^3$ einen Rest der Formel

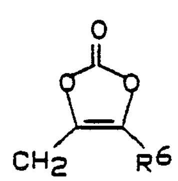

bedeutet, und
e) $R^4$ und $R^5$ zusammen mit den sie tragenden Atomen ein bi- oder tricyclisches heterocyclisches Ringsystem mit 5 bis 15 Ring-C-Atomen bilden;
oder deren physiologisch verträglichen Salze mit Säuren und Basen.

3. Verfahren gemäß Anspruch 1 oder 2, dadurch gekennzeichnet, daß eine Verbindung der Formel I hergestellt wird, in welcher
n = 1 oder 2 ist

R = Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C-Atomen,
das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-$(C_1-C_4)$-alkyl,
$(C_1-C_4)$-Alkanoylamino-$(C_1-C_4)$alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4)$-alkylthio,
das im Arylteil wie oben bei Aryl beschrieben substituiert sein kann,
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_6-C_{12})$-Aryloxy-$(C_1-C_4)$-alkyl,
das im Arylteil wie oben bei Aryl beschrieben substituiert sein kann oder
$(C_6-C_{12})$-Aryl-$(C_1-C_4)$-alkoxy,
das im Arylteil wie oben bei Aryl beschrieben substituiert sein kann,

$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6 - 12 C-Atomen, das wie oben bei R beschrieben substituiert sein kann, $(C_6-C_{12})$-Aryl-$(C_1-C_4)$-alkyl oder $(C_7-C_{13})$-Aroyl-($C_1$ oder $C_2$)-alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder
1 bis 4 Ringatome Stickstoffatome darstellen, das wie das vorstehende Aryl substituiert sein kann oder
die gegebenenfalls geschützte Seitenkette einer natürlichen vorkommenden α-Aminosäure $R^1$-$CH(NH_2)$-COOH bedeuten,
$R^2$ und $R^3$ gleich oder verschieden sind und
Wasserstoff,
Alkyl mit 1 - 6 C-Atomen,

Alkenyl mit 2 - 6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6 - 12 C-Atomen,
($C_6$-$C_{12}$)-Aryl-($C_1$-$C_4$)-alkyl,
($C_3$-$C_9$)-Cycloalkyl,
($C_3$-$C_9$)-Cycloalkyl-($C_1$-$C_4$)-alkyl,
Phthalidyl oder
einen Rest der Formel

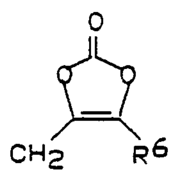

bedeuten, worin $R^6$ Wasserstoff, ($C_1$-$C_6$)-Alkyl oder Aryl mit 6 - 12 C-Atomen ist und wobei mindestens einer der Reste $R^2$ oder $R^3$ einen Rest der Formel

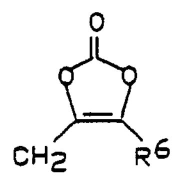

bedeutet, und
$R^4$ und $R^5$ die oben angegebene Bedeutung haben sowie deren physiologisch verträglichen Salze mit Säuren und Basen.

4. Verfahren gemäß einem oder mehreren der Ansprüche 1 bis 3, dadurch gekennzeichnet, daß eine Verbindung der Formel I hergestellt wird, in welcher
n = 1 oder 2 ist,
R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxy-carbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono- oder disubstituiert oder im Falle von Methoxy, trisubstituiert sein kann, bedeutet,

$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)- Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl, ($C_6$-$C_{12}$)-Aryl-($C_1$-$C_4$)-alkyl, ($C_1$-$C_5$)-Alkanoyloxy-($C_1$-$C_4$)-alkyl, ($C_1$-$C_6$)-Alkoxycarbonyloxy-($C_1$-$C_4$)-alkyl, ($C_7$-$C_{13}$)-Aroyloxy-($C_1$-$C_4$)-alkyl, ($C_6$-$C_{12}$)-Aryloxycarbonyloxy-($C_1$-$C_4$)alkyl, Phthalidyl oder einen Rest der Formel

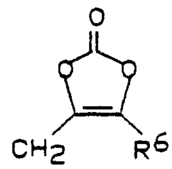

bedeutet,
worin $R^6$ Wasserstoff, ($C_1$-$C_6$)-Alkyl oder Aryl mit 6 - 12 C-Atomen ist, wobei mindestens einer der Reste $R^2$ oder $R^3$ einen Rest der Formel

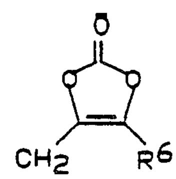

bedeutet,
insbesondere aber Wasserstoff, ($C_1$-$C_4$)-Alkyl, Benzyl, Acetoxymethyl, Propionyloxymethyl, Isopropionyloxymethyl, n-Butyryloxymethyl, Pivaloyloxymethyl, Isovaleroyloxymethyl, 1-Acetoxyethyl, 1-(n-Propionyloxy)-ethyl, 1-Acetoxypropyl, 1-(Methoxycarbonyloxy)ethyl, 1-(Ethoxycarbonyloxy)ethyl, 1-(Isopropoxycarbonyloxy)-ethyl, 1-(Cyclohexyloxycarbonyloxy)ethyl, Methoxycarbonyloxymethyl, Phthalidyl, (5-Methyl-1,3-dioxolen-2-on-4-yl)methyl, (5-tert. Butyl-1,3-dioxolen-2-on-4-yl)methyl oder (5-Phenyl-1,3-dioxolen-2-on-4-yl)methyl und
$R^4$ und $R^5$ die oben angegebene Bedeutung haben, oder deren physiologisch verträglichen Salze mit Säuren und Basen.

5. Verfahren zur Herstellung einer Verbindungen der Formel V

$$R-(CH_2)_n - \overset{*}{C}H(COOR^2) - OSO_2CF_3 \qquad (V)$$

worin R und n die gleiche Bedeutung wie in Formel I besitzen und worin $R^2$ einen Rest der Formel

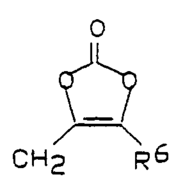

worin $R^6$ Wasserstoff, ein aliphatischer Rest mit 1 - 6 C-Atomen, oder ein gegebenenfalls substituierter aromatischer Rest mit 6 - 12 C-Atomen ist, bedeutet, dadurch gekennzeichnet, daß man eine Verbindung der Formel IX

$$R-(CH_2)_n - CH(COOH) - OH \qquad (IX)$$

worin R und n die gleiche Bedeutung wie in Formel I besitzen, mit einer Verbindung der Formel III unter den Bedingungen einer nucleophilen Substitution umsetzt und anschließend die Hydroxygruppe in die

$-OSO_2CF_3$-Gruppe überführt.

6. Verfahren zur Herstellung eines pharmazeutischen Mittels enthaltend eine Verbindung gemäß einem oder mehrerer der Ansprüche 1 bis 4, dadurch gekennzeichnet, daß man eine Verbindung der Formel I oder deren physiologisch verträgliches Salz und einen physiologisch annehmbaren Träger gegebenenfalls zusammen mit weiteren Zusatz- oder Hilfsstoffen in eine geeignete Darreichungsform bringt.